(19) Europäisches Patentamt
European Patent Office
Office européen des brevets

(11) EP 1 229 892 B1

(12) # FASCICULE DE BREVET EUROPEEN

(45) Date de publication et mention de la délivrance du brevet:
**15.09.2004 Bulletin 2004/38**

(21) Numéro de dépôt: **01976385.3**

(22) Date de dépôt: **09.10.2001**

(51) Int Cl.[7]: **A61K 7/13**, A61K 7/06, A61K 7/48, A61K 7/42

(86) Numéro de dépôt international:
**PCT/FR2001/003110**

(87) Numéro de publication internationale:
**WO 2002/030375 (18.04.2002 Gazette 2002/16)**

(54) **COMPOSITION DE COLORATION, PROCEDE D'OBTENTION ET UTILISATION POUR LA COLORATION DE LA PEAU ET/OU DES MATIERES KERATINIQUES**

FÄRBUNGSZUSAMMENSETZUNG, VERFAHREN ZUR HERSTELLUNG UND VERWENDUNG ZUM FÄRBEN DER HAUT UND/ODER DER KERATINISCHEN FASERN

DYEING COMPOSITION, METHOD FOR OBTAINING SAME AND USE FOR COLOURING THE SKIN AND/OR KERATINOUS FIBRES

(84) Etats contractants désignés:
**AT BE CH CY DE DK ES FI FR GB GR IE IT LI LU MC NL PT SE TR**

(30) Priorité: **09.10.2000 FR 0012894**
**09.10.2000 FR 0012895**

(43) Date de publication de la demande:
**14.08.2002 Bulletin 2002/33**

(73) Titulaire: **L'OREAL**
**75008 Paris (FR)**

(72) Inventeur: **PRUCHE, Françis**
**F-60300 Senlis (FR)**

(74) Mandataire: **Catherine, Alain**
**Cabinet Harlé & Phélip**
**7, rue de Madrid**
**75008 Paris (FR)**

(56) Documents cités:
**GB-A- 917 840** **GB-A- 2 024 623**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 229 892 B1

Printed by Jouve, 75001 PARIS (FR)

## Description

La présente invention concerne d'une manière générale une composition de coloration, en particulier pour la coloration de la peau et/ou des fibres kératiniques, un procédé d'obtention d'une telle composition de coloration et son utilisation pour la coloration de la peau et/ou des fibres kératiniques.

Selon un mode de réalisation, la présente invention concerne plus particulièrement une composition de coloration de la peau qui, après application sur celle-ci, lui confère un bronzage artificiel homogène et de préférence d'une teinte proche de celle d'un bronzage naturel.

Dans le domaine de la coloration de la peau et/ou des fibres kératiniques telles que les cheveux, les cils, les sourcils et les poils, on utilise à l'heure actuelle des catalyseurs enzymatiques pour activer la coloration de précurseurs de coloration. Ainsi, on active la coloration de polyphénols par oxydation en présence de polyphénoloxydase naturelle. A titre d'exemple, de la catéchine, en présence de polyphénoloxydase naturelle, donne une coloration jaune orangée et les dihydroxyphénylanaline (L. DOPA) donne de la mélanine. L'avantage principal de ces catalyseurs enzymatiques consiste en l'obtention de pigments de couleurs et de nuances originales, sans utilisation de composés oxydants. Cependant, l'inconvénient majeur de ce procédé de coloration est l'utilisation d'enzymes, pour lesquels les risques toxicologiques, la stabilité dans les compositions, la reproductibilité, le prix et l'immobilisation souvent nécessaire sont des facteurs qui limitent grandement leurs applications.

D'autre part, ces catalyseurs sont de nature protéique et l'utilisation de protéines n'est pas sans risque pour une utilisation en cosmétologie ou en dermatologie, notamment en raison des réactions de sensibilisation.

L'utilisation de catalyseurs enzymatiques dans les préparations cosmétiques du type auto-bronzant ne permet pas toujours une coloration homogène de la peau. L'application sur l'ensemble du corps de compositions contenant de la dihydroxyacétone (ou DHA), typiquement utilisée dans ce type d'application, est longue et fastidieuse, et l'obtention d'une coloration homogène est difficile.

Dans le domaine des crèmes bronzantes et autobronzantes, une amélioration a été obtenue en utilisant à la place des catalyseurs enzymatiques, des catalyseurs chimiques. Ainsi, la demande WO 92/20321 A décrit une crème favorisant le brunissement de peaux claires lors d'une exposition au soleil ou à des rayons UVB, dont la composition comprend un écran solaire, un milieu physiologiquement acceptable et une pseudocatalase. La pseudocatalase est un complexe de coordination d'un métal de transition dont le métal est Cu(I), Fe(II) ou Mn(II) et le ligand du bicarbonate. Par pseudocatalase, on entend un composé physiologiquement acceptable qui catalyse la dismutation de $H_2O_2$ in vivo de manière analogue à une catalase.

Pour le traitement de la dépigmentation de la peau liée à des blocages de la transformation de la tyrosine en mélanine, comme par exemple le vitiligo, la demande WO 92/20354 décrit une composition contenant, dans un milieu physiologiquement acceptable, une pseudocatalase.

Cette pseudocatalase est un complexe de coordination de Fe(II), Cu(I) ou Mn(II), le ligand étant du bicarbonate.

L'article de K. Schallreuter (" Pseudocatalase is a bis-manganese III-EDTA-(HC03)2 complex activated by UVB or natural sun ; J Investing Dermator Symp Proc 1999 Sep ; 451° ; 91-6) mentionne l'utilisation d'un mélange d'hydrogénocarbonate de sodium et de manganèse ayant une activité pseudocatalase pour le traitement du vitiligo. Cependant, il n'y a pas, dans l'ensemble de ces documents, d'indication concernant la coloration.

Dans le domaine de la coloration capillaire, le brevet européen EP 621029 A décrit une composition comprenant du chlorite de sodium, un sel hydrosoluble de Fe, Mn ou Cu, ou un chélate de ce sel et un précurseur de colorant d'oxydation.

La coloration des cheveux nécessite l'emploi de combinaisons $H_2O_2$ ammonium ou amine.

Il existe donc un besoin pour des compositions de coloration, en particulier pour la coloration de la peau et / ou des fibres kératiniques, ne nécessitant pas l'utilisation de systèmes enzymatiques.

La demanderesse a trouvé, de manière tout à fait surprenante, qu'il était possible d'obtenir des compositions de coloration comprenant au moins un précurseur de colorant susceptible de se colorer, en présence d'oxygène, par oxydation au moyen d'un système non enzymatique (oxydase), et éventuellement au moins un acide aminé comportant au moins un groupe thiol (SH), en remplaçant le système enzymatique par un système purement chimique.

Ainsi, le système catalytique chimique de l'invention se comporte comme une pseudo-oxydase capable de mimer l'activité oxydase sans les inconvénients liés à l'emploi d'un système enzymatique.

La présente invention a donc pour objet une composition de coloration, en particulier pour la coloration de la peau et/ou des fibres kératiniques, qui ne nécessite pas la présence d'enzymes.

La présente invention concerne également un procédé pour révéler la coloration d'une composition de base peu ou pas colorée comprenant au moins un précurseur de colorant par oxydation et éventuellement au moins un acide aminé comportant au moins un groupe thiol qui consiste à ajouter à la composition de base un système catalytique purement chimique et à mettre la composition de base additionnée du système catalytique en présence d'un milieu oxydant tel qu'un milieu contenant de l'oxygène.

La présente invention a encore pour objet un procédé d'obtention d'une composition de coloration telle que définie ci-dessus.

La présente invention concerne en outre un procédé de coloration de la peau et/ou des fibres kératiniques utilisant une composition telle que définie ci-dessus.

Enfin, la présente invention concerne des conditionnements et formes galéniques de la composition de coloration ou de constituants de la composition de coloration selon l'invention.

Dans la présente invention on entend par cosmétique un produit faisant appel aux sens ayant un aspect, un toucher, une odeur et un goût agréable.

La composition pour la coloration de la peau et/ou des fibres kératiniques selon l'invention comprend, dans un milieu physiologiquement acceptable, une quantité efficace d'au moins un précurseur de colorant choisi parmi les composés comportant au moins un cycle aromatique ayant au moins deux groupes hydroxyle (OH) portés par deux atomes de carbone consécutifs du cycle aromatique et une quantité efficace d'un système catalytique comprenant un premier constituant choisi parmi les sels et oxydes de Mn(II), et/ou Zn(II) et leurs mélanges et un second constituant choisi parmi les hydrogénocarbonates alcalins, les hydrogénocarbonates alcalino-terreux et leurs mélanges, les proportions du premier constituant et du second constituant étant telles que :

$$\frac{[Mn(II)]}{[HCO_3]} \leq 1 \text{ avec } [Mn(II)] \neq 0$$

$$\frac{[Zn(II)]}{[HCO_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et [$HCO_3$] représentent respectivement les concentrations molaires en Mn(II), Zn(II) et $HCO_3$ dans la composition.

Généralement, le rapport $\frac{[Mn(II)]}{[HCO_3]}$ varie de $10^{-5}$ à $10^{-1}$, de préférence de $10^{-3}$ à $10^{-2}$ et est typiquement de l'ordre de $5.10^{-3}$.

Dans le cas de Zn(II), le rapport $\frac{[Zn(II)]}{[HCO_3]}$ est en général d'un ordre de 10 à 100 fois supérieur au rapport dans le cas de Mn(II).

Typiquement, ce rapport est de $10^{-4}$ ou plus, de préférence $10^{-3}$ ou plus, et de préférence de l'ordre de $5.10^{-1}$.

Dans le cas d'un mélange de Mn(II) et Zn(II), le rapport varie généralement de $10^{-5}$ à $10^{-1}$, de préférence $10^{-3}$ à $10^{-2}$, ce rapport étant choisi plus élevé lorsque la proportion de Zn(II) dans le mélange s'accroît.

Parmi les sels de Mn(II) et Zn(II) convenant pour la présente invention, on peut citer les chlorure, fluorure, iodure, sulfate, acétate phosphate, nitrate et perchlorate, les sels d'acides carboxyliques et leurs mélanges.

A titre d'exemple, on peut citer le chlorure de manganèse, le carbonate de manganèse (par exemple rhodochrosite), le difluorure de Mn(II), l'acétate de Mn(II) tétrahydraté, le lactate de Mn(II) trihydraté, le phosphate de Mn (II), l'iodure de Mn(II), le nitrate de Mn(II) trihydraté, le bromure de Mn(II) et le perchlorate de Mn(II) tétrahydraté, et le sulfate de Mn(II) monohydraté.

Les sels particulièrement préférés sont $MnCl_2$ et $ZnCl_2$.

Les sels d'acides carboxyliques incluent également des sels d'acides carboxyliques hydroxylés tels que le gluconate.

Parmi les hydrogénocarbonates alcalins et alcalino-terreux, on peut citer les hydrogénocarbonates de Na, K, Mg, Ca et leurs mélanges, préférentiellement l'hydrogénocarbonate de Na.

Comme indiqué précédemment, le système catalytique chimique selon l'invention constitue une pseudo-oxydase en ce qu'il oxyde les polyphénols, en présence d'oxygène, comme le ferait un catalyseur enzymatique naturel ayant une activité polyphénoloxydase.

Par contre, le système catalytique selon l'invention n'a pas d'activité pseudocatalase en ce sens qu'il ne provoque pas la dismutation du peroxyde d'hydrogène à 0,3 % en poids (soit 1 volume d'oxygène).

En outre, l'activité pseudo-oxydase est liée à l'emploi du système catalytique selon l'invention. Ainsi, chacun des constituants du système catalytique pris séparément n'a pas d'activité pseudo-oxydase. De même que le remplacement du sel de Mn(II) ou Zn(II) par un autre sel, Fe, Cu ou même Mn(III) ne conduit pas à un système catalytique ayant une activité pseudo-oxydase.

Les précurseurs de colorant des compositions de l'invention sont des composés ou mélanges de composés

comprenant au moins un cycle aromatique, de préférence un cycle benzénique, comportant au moins deux groupes hydroxyles (OH) portés par deux atomes de carbone consécutifs du cycle aromatique.

Le cycle aromatique peut être un cycle aromatique condensé contenant éventuellement un ou plusieurs hétéroatomes, tel que le naphtalène, le tétrahydronaphtalène, l'indane, l'indène, l'anthracène, le phénanthrène, l'indole, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine.

Les précurseurs de colorant selon l'invention peuvent être représentés par la formule :

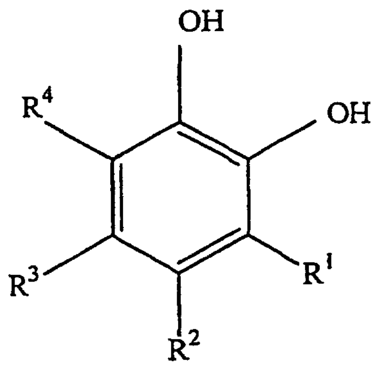

(I)

dans laquelle les substituts $R^1$ à $R^4$, identiques ou différents, représentent un atome d'hydrogène, un radical halogène, hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical contenant un ou plusieurs atomes de silicium, où deux des substituants $R^1$ à $R^4$ forment conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

Les cycles saturés ou insaturés, éventuellement condensés, peuvent être aussi éventuellement substitués.

Les radicaux alkyles sont généralement les radicaux alkyles en $C_1$-$C_{10}$, de préférence les radicaux alkyles en $C_1$-$C_6$, tels que méthyle, éthyle, propyle, butyle, pentyle et hexyle.

Les radicaux alcoxy sont en général les radicaux alcoxy en $C_1$-$C_{20}$, tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle.

Les radicaux cycloalkyles sont en général les radicaux cycloalkyles en $C_4$-$C_8$, de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxyle, amine et cétone.

Les radicaux alcényles sont de préférence des radicaux en $C_1$-$C_{20}$, tels que éthylène, propylène, butylène, pentylène, méthyl-2-propylène et décylène.

Les radicaux contenant un ou plusieurs atomes de silicium sont de préférence des radicaux polydiméthylsiloxane, polydiphenylsiloxane, polydiméthylphénylsiloxane, stéraoxydiméthicone.

Les radicaux hétérocycliques sont en général des radicaux comprenant un ou plusieurs hétéroatomes choisis parmi O, N et S, de préférence O ou N, éventuellement substitués par un ou plusieurs groupes alkyles, alcoxy, acide carboxylique, hydroxyle, amine ou cétone.

Parmi les radicaux hétérocyliques préférés, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH.

Les précurseurs de colorant préférés sont :

- les flavanols comme la catéchine et le gallate d'épichatéchine,
- les flavonols comme la quercétine,
- les anthocyanidines comme la péonidine,

- les anthocyanines, par exemple l'oenine,
- les hydroxybenzoates, par exemple l'acide gallique,
- les flavones comme la lutéoline,
- les iridoïdes comme l'oleuropéine,

ces produits pouvant être osylés (par exemple glucosylés) et /ou sous forme d'oligomères (procyanidines) ;

- les hydroxystilbènes, par exemple le tétrahydroxy-3,3',4,5'-stilbène, éventuellement osylés (par exemple glucosylés) ;
- la 3,4-dihydroxyphénylalanine et ses dérivés ;
- la 2,3-dihydroxyphenylalanine et ses dérivés ;
- la 4,5-dihydroxyphenylalanine et ses dérivés ;
- le 4,5-dihydroxyindole et ses dérivés ;
- le 5,6-dihydroxyindole et ses dérivés ;
- le 6,7-dihydroxyindole et ses dérivés ;
- le 2,3-dihydroxyindole et ses dérivés ;
- les dihydroxycinnamates tels que l'acide caféique et l'acide chlorogénique ;
- les hydroxycoumarines ;
- les hydroxyisocoumarines ;
- les hydroxycoumarones;
- les hydroxyisocoumarones;
- les hydroxychalcones ;
- les hydroxychromones ;
- les anthocyanes ;
- les quinones ;
- les hydroxyxanthones ; et
- les mélanges de ceux-ci.

Lorsque les précurseurs de colorant présentent des formes D et L, les deux formes peuvent être utilisées dans les compositions selon l'invention.
En faisant varier la nature des différents précurseurs de colorant et leurs proportions dans la composition, on peut faire varier la couleur de la composition de coloration finale. On obtient ainsi une palette de couleurs.
Par exemple, avec un ratio 1/10 d'acide chlorogénique et de catéchine, on obtient une coloration marron claire et avec un ratio 1/1 une coloration acajou.
Les polymères formés en particulier avec la catéchine, l'acide gallique et leurs dérivés (tannins) ont des propriétés antimicrobiennes par emprisonnement des microorganismes lors de la polymérisation. Ces tannins ont également des propriétés astringentes intéressantes pour la peau.
Les précurseurs de colorants peuvent être des extraits de plantes, fruits, agrumes, légumes et des mélanges de ces extraits, qui contiennent de nombreux polyphénols tels que définis précédemment.
Parmi les extraits de plantes, on peut citer les extraits de rose et de thé.
Parmi les extraits de fruits, on peut citer les extraits de pomme, de raisin (en particulier de pépins de raisin) et de banane.
Parmi les extraits de légumes, on peut citer l'extrait de pomme de terre.
On peut également utiliser des mélanges d'extraits de plantes et/ou de fruits tels que des mélanges d'extraits de pomme et de thé et des mélanges d'extraits de raisin et de pomme.
Suivant les parties de fruits utilisés, par exemple pulpe ou pépins de raisin, la coloration obtenue est différente.
La quantité de précurseur de colorant dans la composition finale doit être suffisante pour obtenir une coloration visible. Cette quantité peut varier dans de larges mesures en fonction de la nature du précurseur et de l'intensité voulue pour la coloration.
En général, on obtiendra une coloration convenable lorsque la quantité de précurseur de colorant est telle que la teneur en précurseur de colorant dans la composition de coloration finale est d'au moins 10 micromoles par millilitre de composition finale.
Dans un mode de réalisation particulier, les compositions selon l'invention contiennent également une quantité efficace d'au moins un acide aminé comportant au moins un groupe thiol.
Dans ce mode de réalisation, les précurseurs de colorants d'oxydation préférés selon l'invention sont les dihydroxyphénylalanines et leurs dérivés et les dihydroxyindoles et leurs dérivés.
L'acide aminé comporte au moins un groupe thiol (SH) et de préférence un seul groupe thiol, cet acide aminé pouvant être sous la forme d'un chlorhydrate.

Les acides aminés préférés selon l'invention sont les acides aminés qui contiennent une fonction amine en position α par rapport à une fonction acide carboxylique.

Les acides aminés préférés peuvent être représentés par la formule

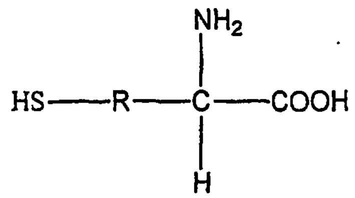

(II)

dans laquelle -R est un radical hydrocarboné divalent, linéaire ou ramifié, par exemple en $C_1$-$C_{10}$, de préférence en $C_1$-$C_6$, tel qu'un radical méthylène, éthylène, butylène, éthylidène, propylidène, un radical cyclique saturé divalent, éventuellement substitué, par exemple en $C_4$-$C_8$, un groupe aromatique divalent, éventuellement substitué, tel qu'un radical phénylène, tolylène, xylylène.

Parmi les acides aminés préférés pour les compositions de l'invention, on peut citer la cystéine et ses dérivés, en particulier la L-cystéine et le chlorhydrate de L-cystéine, et le glutathion et ses dérivés.

Les proportions relatives d'acide aminé et de précurseur de colorant d'oxydation dans les compositions de l'invention peuvent varier dans de larges mesures en fonction de la coloration voulue. Généralement, le rapport molaire acide aminé/précurseur de colorant variera de 0,001 à 50, de préférence de 0,01 à 5, et mieux de 0,05 à 2,5.

En général, la teneur en acide aminé à groupe thiol, dans la composition finale est d'au moins 0,01 micromole par millilitre, de préférence au moins 0,1 micromole/ml.

Bien évidemment, les doses des constituants des compositions de l'invention sont non toxiques et compatibles avec la cosmétique.

En faisant varier la nature des précurseurs de colorant et des acides aminés de la composition et la proportion relative d'acide aminé et de précurseur de colorant, on peut obtenir toute une palette de teintes et en particulier des teintes proches de celles du bronzage naturel.

Ainsi, l'association de la cystéine et de dihydroxyindole permet d'obtenir une nuance marron, plus proche de celle d'un bronzage naturel que l'emploi du seul dihydroxyindole qui conduit à une couleur uniquement noire.

Cette association permet d'avoir une coloration naturelle de la peau et du cheveu qui est un mélange d'eumélanine et de phéomélanine en proportions variables.

Les compositions de ce mode particulier de l'invention peuvent également conduire à des films colorés qui présentent une certaine transparence. Cette caractéristique, alliée au fait que ces compositions permettent d'obtenir une teinte proche du bronzage naturel, rend ces compositions particulièrement intéressantes pour l'application au bronzage de la peau, permettant de combiner bronzage artificiel et bronzage naturel tout en obtenant une teinte homogène.

Le milieu physiologiquement acceptable est un milieu solide ou liquide ne nuisant pas à la propriété de coloration des précurseurs ni à l'effet catalytique du système catalytique.

Le milieu physiologiquement acceptable est de préférence un milieu solubilisant du précurseur de colorant et de l'acide aminé et à propriété bactériostatique.

Parmi les solvants des précurseurs convenant pour la formulation des compositions selon l'invention, on peut citer l'eau déminéralisée, les alcools, les solvants polaires et leurs mélanges.

Les alcools sont de préférence des alcanols inférieurs ($C_1$-$C_6$) tels que l'éthanol et l'isopropanol et les alcanediols tels que l'éthylèneglycol, le propylène glycol et le pentane diol.

Parmi les solvants polaires, on peut citer les éthers, les esters (en particulier les acétates), le diméthylsulfoxyde (DMSO), la N-méthylpyrrolidone (NMP), les cétones (en particulier l'acétone) et leurs mélanges.

Le milieu physiologiquement acceptable comprend de l'eau déminéralisée de préférence ayant une conductivité à température (25°C) inférieure ou égale à 25 mΩ, par exemple l'eau fabriquée et/ou distribuée par la société Millipore sous la dénomination milli Q, ou un mélange eau/alcool, en particulier eau/éthanol.

La quantité d'alcool dans le mélange eau/alcool peut représenter jusqu'à 80% en poids du mélange eau/alcool, de préférence 1 à 50% en poids et mieux 5 à 20% en poids.

Le milieu physiologiquement acceptable peut être un milieu solide tel qu'un excipient pour la formulation de galets et comprimés, en particulier effervescents.

Les compositions selon l'invention peuvent également comprendre tout adjuvant classique, en proportion usuelle, qui ne nuit pas aux propriétés recherchées, en particulier à l'effet colorant des compositions.

Les compositions de l'invention peuvent par exemple comporter des filtres solaires et de protection contre les UV, tels que les filtres organiques comme le benzophénone, le benzylidène, les dérivés de triazine, les dérivés de l'hydroxyphényl benzotriazole, les dérivés de l'acide cinnamique, les dérivés d'oxybenzone, l'octocrylène, les dérivés de benzilidène-camphre et les filtres minéraux tels que ZnO, $TiO_2$.

Lorsque les compositions selon l'invention comportent des pigments et des colorants classiques, elles peuvent être utilisées pour obtenir des produits tels que des fonds de teint dont l'action colorante apportée à la peau par les pigments et/ou colorants est prolongée dans le temps. En effet, par suite du frottement notamment des joues et du cou, un fond de teint classique et par suite son effet colorant tend à disparaître. L'utilisation d'une composition selon l'invention dans une formulation de fond de teint permettrait donc de pallier cet effet.

De préférence, les compositions selon l'invention sont exemptes d'agents de chélation des sels de Mn(II) et/ou Zn(II) utilisés, car ces agents tendent à inhiber l'oxydation des précurseurs de colorant.

Pour révéler la coloration des compositions suivant l'invention, il suffit de mettre la composition contenant au moins un précurseur de colorant et une quantité efficace du système catalytique selon l'invention, en présence d'un milieu oxydant tel qu'un milieu contenant de l'oxygène (par exemple l'oxygène de l'air).

Les compositions selon l'invention sont utiles pour la coloration de la peau et/ou des fibres kératiniques tels que les cheveux, les cils, les sourcils et les poils. Elles peuvent également être utilisées pour la coloration d'aliments.

Pour la coloration de la peau et des fibres kératiniques, différents procédés d'application des compositions selon l'invention peuvent être utilisés.

Selon un premier procédé, on applique sur la peau ou les fibres kératiniques, en présence d'oxygène par exemple l'oxygène de l'air, une composition comprenant tous les ingrédients de la composition, c'est-à-dire à la fois le ou les précurseurs de colorant et le système catalytique. La coloration est alors révélée directement sur la peau ou les fibres kératiniques. On peut en variante révéler la coloration de la composition avant son application sur la peau ou les fibres kératiniques et appliquer la composition colorée sur la peau ou les fibres kératiniques.

Selon un second procédé, on peut en premier lieu appliquer sur la peau ou les fibres kératiniques un film d'une composition de base d'un ou plusieurs précurseurs de colorant dans un milieu physiologiquement acceptable, puis sur le film de composition de base, un film du système catalytique dans un milieu physiologiquement acceptable, qui en présence d'oxygène, révélera la coloration de la composition de base.

On peut bien évidemment inverser l'ordre d'application des films.

L'application des films peut se faire par tout moyen connu, en particulier par pulvérisation.

Une fois la composition selon l'invention appliquée sur la peau et/ou les fibres kératiniques, on peut appliquer une solution d'un ou plusieurs orthodiphénols, de préférence différents de ceux de la composition appliquée, et, dans la mesure où le système catalytique initialement présent n'a pas été totalement consommé, l'apport de cet (ou ces) orthodiphénol(s) supplémentaire(s) augmentera la rémanence de la coloration et/ou selon le (ou les) orthodiphénol(s) utilisé(s) augmentera l'intensité.

Les compositions de l'invention peuvent, en fonction du choix des précurseurs de colorant, constituer des compositions de bronzage artificiel qui, lorsqu'on y a incorporé des filtres solaires, confèrent à la peau un aspect artificiellement bronzé, tout en permettant une exposition sans risque au soleil ou aux UV pour un bronzage naturel.

Les compositions de l'invention peuvent également permettre de masquer des défauts de pigmentation tels que le vitiligo, le masque de grossesse, ainsi que des imperfections de la peau comme les taches de vieillissement et la couperose.

La coloration de la composition peut être déterminée par le choix des précurseurs de colorant et/ou de l'acide aminé à groupe thiol en fonction du défaut à masquer.

Les compositions de l'invention présentent l'avantage de ne pas nécessiter l'emploi de peroxyde d'hydrogène.

Les compositions selon l'invention peuvent se présenter et être conditionnées sous différentes formes.

Selon une première réalisation, les compositions selon l'invention peuvent être conditionnées sous forme d'aérosol à un seul compartiment dans lequel se trouvent la composition renfermant le ou les précurseurs de colorant, éventuellement le ou les acides aminés, et le système catalytique, et un gaz propulseur inerte classique tel que de l'azote, un hydrocarbure saturé comme l'isopropane ou un hydrocarbure fluoré, par exemple un Fréon®.

Dans une seconde réalisation, la composition selon l'invention peut être conditionnée sous forme d'un kit comportant deux conteneurs distincts, l'un pour la composition de base contenant le ou les précurseurs de colorant et éventuellement le ou les acides aminés, l'autre pour le système catalytique, la composition de base et le système catalytique étant mélangés ou appliqués successivement au moment de l'emploi.

Dans une troisième réalisation, la composition peut être contenue dans un système à pompe à un seul compartiment, sans reprise d'air, ou dans un système à pompe à deux compartiments, la composition de base étant dans un compartiment et le système catalytique dans l'autre.

Dans une quatrième réalisation, la composition selon l'invention peut se présenter sous forme de galets, en particulier pour le bain. Chaque galet peut comporter, mélangé à un excipient , le ou les précurseurs de coloration, éventuellement le ou les acides aminés, et le système catalytique, l'excipient empêchant la réaction en présence

d'oxygène ou le ou les précurseurs de colorant et éventuellement le ou les acides aminés et le système catalytique sont contenus dans des galets distincts.

En délitant soit le galet unique soit un galet de chacun des constituants dans de l'eau, par exemple l'eau d'un bain, on réalise la composition colorante selon l'invention.

Les galets, comme cela est classique, peuvent être des galets effervescents .

L'excipient utilisé peut être tout excipient classique tel qu'un mélange de talc, de stéarate (en particulier de magnésium), d'acide citrique et/ou tartrique, et d'hydrogèno carbonate alcalin et / ou alcalino terreux.

La quantité d'acide citrique et/ou tartrique présente doit être telle qu'il n'y ait pas une neutralisation de l'hydrogénocarbonate résultant en un manque d'hydrogénocarbonate libre par rapport au Mn(II) et/ou Zn(II).

D'autre part, comme l'eau, en particulier l'eau du robinet et certaines eaux de source ou minérales, contiennent généralement du manganèse (II), il est parfois suffisant de mettre dans l'eau le seul galet contenant de l'hydrogénocarbonate et le ou les précurseurs de coloration, la teneur en Mn(II) du système catalytique étant alors fournie par le Mn(II) présent dans l'eau.

De même, certains extraits végétaux (par exemple des extraits de feuilles de thé) peuvent contenir des quantités importantes de manganèse (II). En fonction de ces teneurs, un ajustement des concentrations du système catalytique est effectué de manière à avoir un résultat satisfaisant.

Bien évidemment, on peut faire varier l'intensité de la coloration en délitant plusieurs galets dans l'eau.

D'autre part, la vitesse de coloration peut être accélérée en ajoutant à la composition un composé ou une formulation de composés engendrant de l'oxygène, par exemple par contact avec de l'eau. Ainsi, on peut incorporer un tel composé ou formulation, par exemple du peroxyde de sodium, dans un galet.

Les exemples suivants illustrent la présente invention. Dans les exemples, sauf indication contraire, tous les pourcentages et parties sont exprimés en poids:

EXEMPLE 1

On a préparé plusieurs formulations de composition de coloration en faisant varier le rapport molaire [$MnCl_2$] / [$NaHCO_3$] du système catalytique. Les formulations sont données dans le tableau I ci-dessous :

TABLEAU I

| Formulation N° | Précurseur de coloration | | Milieu physiologiquement acceptable | Système catalytique | | |
|---|---|---|---|---|---|---|
| | Nature | Concentration (mM/l) | | Ratio $MnCl_2$ $NaHCO_3$ | Concentration $MnCl_2$ (mM/l) | Concentration $NaHCO_3$ (mM/l) |
| 1 | Catéchine | 1 | Solution saline avec Tampon phosphate De Dulbecco (PBS) soit en g/l :<br>$KH_2PO_4$ : 0,2<br>$MnCl_2$ : 0,2<br>NaCl : 8,0 et<br>$Na_2HPO_4$ : 1,15<br>et 10 % en poids d'éthanol | $10^{-3}$ | $10^{-2}$ | 10 |
| 2 | Catéchine | 1 | | $2,5.10^{-3}$ | $2,5.10^{-2}$ | 10 |
| 3 | Catéchine | 1 | | $5.10^{-3}$ | $5.10^{-2}$ | 10 |
| 4 | Catéchine | 1 | | $10^{-2}$ | $10^{-1}$ | 10 |
| 5 | L-DOPA | 1 | | $10^{-3}$ | $10^{-2}$ | 10 |
| 6 | L-DOPA | 1 | | $2,5.10^{-3}$ | $2,5.10^{-2}$ | 10 |
| 7 | L-DOPA | 1 | | $5.10^{-3}$ | $5.10^{-2}$ | 10 |
| 8 | L-DOPA | 1 | | $10^{-2}$ | $10^{-1}$ | 10 |

On a mesuré les vitesses maximales (Vmax) de coloration à l'air des formulations ci-dessus. La vitesse maximale est l'augmentation de la millidensité optique (mDo) par minute sur la zone de la cinétique dans laquelle cette vitesse est maximum. Une mesure est réalisée toutes les minutes et la détermination de Vmax s'effectue sur 8 points pendant les 40 premières minutes. Les lectures en densité optique ont été effectuées à 475 nm avec un lecteur de densité optique Labsystem iEMS.

Les résultats sont donnés dans le Tableau II ci-dessous :

TABLEAU II

| Formulation N° | Vmax |
|---|---|
| 1 | 135 |
| 2 | 155,8 |
| 3 | 177,8 |
| 4 | 158 |
| 5 | 38,3 |
| 6 | 48,8 |
| 7 | 61 |
| 8 | 55,8 |

EXEMPLE 2

On a préparé plusieurs échantillons de 500 µl de formulations de composition de coloration selon l'invention contenant un acide aminé à groupe thiol ainsi qu'un échantillon de 500 µl d'une formulation comparative ne contenant pas d'acide aminé à groupe SH.

Les formulations sont données dans le tableau III ci-dessous.

TABLEAU III

| | FORMULATIONS | | | | | |
|---|---|---|---|---|---|---|
| | Comparative A | 9 | 10 | 11 | 12 | 13 |
| L-DOPA | 5 mM/l | 5 mM/l | 5 mM/l | 5 mM/l | 5 mM/l | 5 mM/l |
| L-Cystéine | - | 200 µM/l | 1 mM/l | 2 mM/l | 5 mM/l | 10 mM/l |
| Système catalytique<br>$MnCl_2$<br>$NaHCO_3$<br>EtOH | 0,5 mM/l<br>0,5 M/l<br>10 µl | 0,5 mM/l<br>0,5 M/l<br>10 µl | 0,5 mM/l<br>0,5 M/l<br>10 µl | 0,5 mM/l<br>0,5 M/l<br>10 µl | 0,5 mM/l<br>0,5 M/l<br>10 µl | 0,5 mM/l<br>0,5 M/l<br>10 µl |
| Eau qsp | 500 µl | 500 µl | 500 µl | 500 µl | 500 µl | 500 µl |

La L-DOPA et la L-Cystéine sont introduites dans les formulations sous forme de solutions dans l'éthanol (100 mM/l).

Le système catalytique est introduit sous forme de solution aqueuse (250 µl).

On a déterminé les spectres d'absorption des formulations ci-dessus au moyen d'un spectromètre lambda 2 de Perkin-Elmer.

On voit que l'addition de L-cystéine dans les formulations décale le spectre d'absorption vers la couleur jaune orangée.

EXEMPLE 3

Cet exemple montre l'influence d'un extrait de thé fortement dosé en polyphénols sur la rémanence des produits issus du mélange réactionnel (L-Dopa+L cystéine+ $MnCl_2$ + $ZnCl_2$ + Bicarbonate de sodium + $H_2O$ = composition A).

Pour ce faire on utilise un véhicule (V) qui est un mélange de solvants (éthanol/isopropanol/propylène glycol/urée) (32.3/32.3/32.4/3).

Préparation de la composition A :

On prépare séparement une première solution comprenant :

| | |
|---|---|
| L-Dopa | 200mg |
| L Cystéine | 350mg |
| $MnCl_2$ | 200mg |
| $ZnCl_2$ | 130mg |
| $H_2O$ | qs25 ml |

et une deuxième solution

| | |
|---|---|
| Bicarbonate de sodium | 2.1g |
| $H_2O$ | qs25 ml |

On verse la première solution dans la deuxième solution pour obtenir la composition A.

On ajoute alors au mélange 500mg de peroxyde de sodium.

On laisse à l'air 24H($A_{24H}$) sous agitation puis on conditionne sous argon.

Préparation de l'extrait de thé

On mélange :

| | |
|---|---|
| Green tea 95 | 1 g |
| Ethanol | qsp 10ml |

Essai in vivo :

On réalise plusieurs préparations avec les actifs cités ci-dessus. Le détail des préparations réalisées est indiqué dans le tableau IV ci-après.

La composition A, le véhicule et l'extrait de thé sont mélangés de façon extemporanée avant application sur la peau.

Les quantités présentes dans la préparation extemporanée sont les suivantes :

- Composition A : 1ml
- Véhicule : 1.5mL
- Extrait de thé alcoolique : 100µl

Quand l'extrait de thé n'est pas présent, il est remplacé par le véhicule.

Le site d'application choisi est l'avant- bras. Des carrés de 2.5*2.5 $cm^2$ sont dessinés sur la peau. Les mesures colorimétriques ont été effectuées à l'aide d'un spectrocolorimètre Minolta CR-508d.

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

Le DE exprime les différences de couleur avant et après application des compositions, soit avant bain, soit après bain.

Les mesures colorimétriques sont faites selon la séquence suivante :

- T= 0 mesure de la peau non colorée
- T= 5 minutes, application des compositions à 4mg/$cm^2$
- T=3 heures, lecture des peaux colorés (le temps de 3 H a été choisi de manière arbitraire dans cette expérience préliminaire)
- T=3 heures et cinq minutes, réalisation d'un bain de 1 minute dans de l'eau à 28°C
- T= 3 heures et quinze minutes, séchage à l'air et mesure de la couleur des peaux colorées

TABLEAU IV

| Composition | | | quantité appliquée | Avant bain | | | | Après bain | | | | % perte du DE entre avant et après bain |
|---|---|---|---|---|---|---|---|---|---|---|---|---|
| | | | | L* | a* | b* | DE av bain♦ | L* | a* | b* | DE ap bain♦♦ | |
| V | $A_{24H}$ | Thé | 4mg/cm² | 57,99 | 11,88 | 20,24 | 9,29 | 59,12 | 10,33 | 15,32 | 4,61 | 50,4 |
| V | $A_{24H}$ | | 4mg/cm² | 61,45 | 8,96 | 18,79 | 6,65 | 62,12 | 8,47 | 13,96 | 2,13 | 68,0 |

V : véhicule

A : produits issus du mélange réactionnel (L-Dopa+L cystéine+ $MnCl_2$ + $ZnCl_2$ + Bicarbonate de sodium + $H_2O$)

Thé : extrait de thé (green tea 95)

♦Le DE exprime les différences de couleur avant et après application des produits, avant bain

♦♦Le DE exprime les différences de couleur avant et après application des produits, après bain

- L'ajout de thé à la préparation $A_{24H}$ permet d'obtenir une couleur plus intense et légèrement plus rouge.
- La préparation $A_{24H}$ seule a un % de perte de la couleur assez important (% perte du DE de l'ordre de 68%)
- L'ajout de thé à la préparation $A_{24H}$ permet d'obtenir une diminution de la perte de la couleur (on passe de 68% de perte du DE à 50.4%).

L'ajout de thé permet d'augmenter la rémanence de la couleur des produits issus du mélange réactionnel (L-Dopa+L cystéine+ $MnCl_2$ + $ZnCl_2$ + Bicarbonate de sodium + $H_2O$).

Les compositions de coloration selon l'invention présentent de nombreux avantages dans le domaine de la coloration de la peau et du cheveu. Par exemple, la tenue sur la peau est renforcée par le fait de réaliser la réaction " in situ ". La coloration peut être réalisée avec des extraits naturels contenant des polyphénols et avoir une coloration proche du henné sans l'inconvénient de la présence de naphtoquinones dans ces colorations. Un autre avantage est d'avoir des réactions comme certaines enzymes sans les problèmes liés à leurs utilisations dans lé domaine cosmétique et dermatologique.

Pour le domaine plus biologique la vectorisation de tels actifs est plus facile en raison de la taille et le risque toxicologique nettement moindre que l'usage de protéines. La demi-vie d'un tel mélange est probablement supérieure à une protéine qui subirait sans immobilisation préalable une dégradation rapide par les protéases des tissus.

L'usage d'une composition bronzante pour le bain présente l'intérêt d'éviter au consommateur de s'exposer longuement aux UV pour avoir un résultat (l'association avec une formulation hautement filtrante est donc possible et assure une sécurité plus efficace vis-à-vis des UV).

L'utilisation de l'hydrogénocarbonate de sodium ne semble pas présenter de risques toxicologiques (utilisation autorisée en cosmétique) et la quantité de manganèse est extrêmement faible. Par exemple, pour un bain avec un extrait de thé vert (100g), 20 g d'hydrogénocarbonate de sodium et 100mg de chlorure de manganèse, soit un rapport [Mn(II)/[$HCO_3$] de l'ordre de 1/500, suffisent pour avoir un développement de couleur dans un bain de 100 litres. Certains extraits naturels (par exemple de thé) contiennent du Mn(II) et, dans ce cas, il suffira d'adapter la posologie de la formulation en fonction de la teneur en Mn(II) de l'extrait naturel.

## Revendications

1. Composition pour la coloration de la peau et/ou des fibres kératiniques, **caractérisée en ce qu'**elle comprend, dans un milieu physiologiquement acceptable, une quantité efficace d'au moins un précurseur de colorant choisi parmi les composés comportant au moins un cycle aromatique ayant au moins deux groupes hydroxyles portés par deux atomes de carbone consécutifs du cycle aromatique, facultativement une quantité efficace d'au moins un acide aminé comportant au moins un groupe thiol, et une quantité efficace d'un système catalytique comprenant un premier constituant choisi parmi les sels et oxydes de Mn(II) et/ou de Zn(II) et leurs mélanges et un second constituant choisi parmi les hydrogénocarbonates alcalin, les hydrogénocarbonates alcalino-terreux et leurs mélanges, les proportions du premier et du second constituant étant telles que :

$$\frac{[Mn(II)]}{[HCO_3]} \leq 1 \text{ avec } [Mn(II)] \neq 0$$

$$\frac{[Zn(II)]}{[HCO_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et [$HCO_3$] représentent respectivement les concentrations molaires en Mn(II), Zn(II) et $HCO_3$ dans la composition.

2. Composition selon la revendication 1, **caractérisée en ce que** le rapport $\frac{[Mn(II)]}{[HCO_3]}$ varie de $10^{-5}$ à $10^{-1}$, de préférence de $10^{-3}$ à $10^{-2}$ et mieux est de l'ordre de $5.10^{-3}$.

3. Composition selon la revendication 1 ou 2, **caractérisée en ce que** le rapport $\frac{[Zn(II)]}{[HCO_3]}$ varie de $10^{-4}$ à < 1, de préférence de $10^{-3}$ à < 1, et mieux est de l'ordre de $5.10^{-1}$.

4. Composition selon l'une quelconque des revendications précédentes, **caractérisée en ce que** le rapport $\frac{[Mn(II) + Zn(II)]}{[HCO_3]}$ varie de $10^{-5}$ à $10^{-1}$, de préférence $10^{-3}$ à $10^{-2}$.

5. Composition selon l'une quelconque des revendications précédentes, **caractérisée en ce que** les sels de Mn(II)

et de Zn(II) sont choisis parmi les chlorure, fluorure, iodure, sulfate, phosphate, nitrate, perchlorate, les sels d'acides carboxyliques et leurs mélanges.

6. Composition selon la revendication 5, **caractérisée en ce que** le sel de Mn(II) et/ou de Zn(II) est le chlorure.

7. Composition selon la revendication 5, **caractérisée en ce que** les sels d'acides carboxyliques sont des sels d'acides carboxyliques hydroxylés.

8. Composition selon la revendication 7, **caractérisée en ce que** le sel d'acide carboxylique hydroxylé est le gluconate.

9. Composition selon l'une quelconque des revendications précédentes, **caractérisée en ce que** l'hydrogénocarbonate est choisi parmi l'hydrogénocarbonate de sodium, l'hydrogénocarbonate de potassium, l'hydrogénocarbonate de magnésium, l'hydrogénocarbonate de calcium et leurs mélanges.

10. Composition selon l'une quelconque des revendications précédentes, **caractérisée en ce que** le cycle aromatique comportant au moins deux groupes hydroxyle sur deux atomes de carbone consécutifs du précurseur de colorant est un cycle benzénique ou un cycle aromatique condensé.

11. Composition selon la revendication 10, **caractérisée en ce que** le précurseur de colorant est un composé de formule :

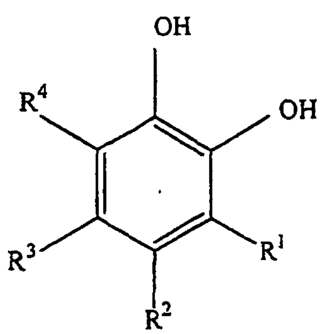

(I)

dans laquelle les substituts $R^1$ à $R^4$, identiques ou différents, représentent un atome d'hydrogène, un halogène, un radical hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical contenant éventuellement un ou plusieurs atomes de silicium, ou deux des substituants $R^1$ à $R^4$ peuvent former conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

12. Composition selon l'une quelconque des revendications 1 à 10, **caractérisée en ce que** le précurseur de colorant est choisi parmi les flavanols, les flavonols, les anthocyanidines, les anthocyanines, les hydroxybenzoates, les flavones, les iridoïdes, ces composés pouvant être éventuellement osylés et/ou sous forme d'oligomères, les hydroxystilbènes éventuellement osylés, la 3,4-dihydroxyphénylalanine et ses dérivés, la 2,3-dihydroxyphénylalanine et ses dérivés, la 4,5-dihydroxyphénylalanine et ses dérivés, le 4,5-dihydroxyindole et ses dérivés, le 5,6-dihydroxyindole et ses dérivés, le 6,7-dihydroxyindole et ses dérivés, le 2,3-dihydroxyindole et ses dérivés, les dihydroxycinnamates, les hydroxycoumarines, les hydroxyisocoumarines, les hydroxycoumarones, les hydroxyisocoumarones, les hydroxychalcones, les hydroxychromones, les anthocyanes, les quinones, les hydroxyxantones, et les mélanges de deux ou plus des composés précédents.

13. Composition selon l'une quelconque des revendications 1 à 10, **caractérisée en ce que** le précurseur de colorant est choisi parmi les extraits de plantes, de fruits, d'agrumes, de légumes et leurs mélanges.

14. Composition selon la revendication 13, **caractérisée en ce que** le précurseur de colorant est choisi parmi les

extraits de thé, de raisin, de pomme, de banane, de pomme de terre et leurs mélanges.

**15.** Composition selon l'une quelconque des revendications précédentes, **caractérisée en ce que** le précurseur de colorant est présent en une quantité d'au moins 10 micromoles par millilitre de composition.

**16.** Composition selon l'une quelconque des revendications précédentes, **caractérisée en ce que** le ou les acides aminés comprenant au moins un groupe thiol sont choisis parmi les acides aminés ayant une fonction amine en position $\alpha$ par rapport à une fonction acide carboxylique.

**17.** Composition selon la revendication 16, **caractérisée en ce que** le ou les acides aminés sont choisis parmi la cystéine et ses dérivés et le glutathion et ses dérivés.

**18.** Composition selon l'une quelconque des revendications précédentes, **caractérisée en ce que** le rapport molaire du (ou des) acide(s) aminé(s) à groupe SH au(x) précurseur(s) de colorant(s) varie de 0,001 à 50, de préférence de 0,01 à 5, et mieux de 0,05 à 2,5.

**19.** Composition selon l'une quelconque des revendications précédentes, **caractérisée en ce que** le précurseur de colorant est une dihydroxyphénylalanine ou un dihydroxyindole et l'acide aminé, la cystéine ou le glutathion.

**20.** Composition selon l'une quelconque des revendications précédentes, **caractérisée en ce que** le milieu physiologiquement acceptable est un milieu solubilisant du précurseur de colorant et de l'acide aminé, de préférence à propriété bactériostatique.

**21.** Composition selon l'une quelconque des revendications précédentes, **caractérisée en ce que** le milieu physiologiquement acceptable comprend un solvant ou un mélange de solvants du précurseur de colorant et de l'acide aminé.

**22.** Composition selon la revendication 21, **caractérisée en ce que** le solvant est choisi parmi l'eau, les alcools, les éthers, le diméthylsulfoxyde, la N-méthylpyrrolidone, les cétones et leurs mélanges.

**23.** Composition selon la revendication 22, **caractérisée en ce que** l'alcool est un alcanol ou un alcanediol.

**24.** Composition selon la revendication 22, **caractérisée en ce que** le solvant est un mélange eau/alcool.

**25.** Composition selon la revendication 24, **caractérisée en ce que** l'alcool représente jusqu'à 80% en poids du mélange, de préférence 1 à 50% en poids et mieux de 5 à 20% en poids.

**26.** Composition selon l'une quelconque des revendications précédentes, **caractérisée en ce qu'**elle est exempte de tout agent de chélation du sel de Mn(II) et/ou Zn(II).

**27.** Composition selon l'une quelconque des revendications précédentes, **caractérisée en ce qu'**elle se présente sous la forme de deux composants séparés, un premier composant comprenant le système catalytique dissous dans un milieu physiologiquement acceptable et un deuxième composant comprenant le précurseur de colorant et éventuellement l'acide aminé à groupe thiol dissous dans un milieu physiologiquement acceptable.

**28.** Composition selon l'une quelconque des revendications 1 à 26, **caractérisée en ce qu'**elle est conditionnée sous forme d'un aérosol ou d'un système à pompe sans reprise d'air.

**29.** Composition selon la revendication 27, **caractérisée en ce qu'**elle est conditionnée dans un système à pompe à deux compartiments distincts, chacun des composants étant contenu séparément dans un des deux compartiments.

**30.** Composition selon l'une quelconque des revendications 1 à 19, **caractérisée en ce qu'**elle est conditionnée sous forme d'un galet.

**31.** Composition selon la revendication 30, **caractérisée en ce que** le comprimé comprend un excipient contenant de l'acide citrique et /ou tartrique en quantité sous stoechiométrique par rapport à l'hydrogénocarbonate alcalin et/ou alcalino terreux.

**32.** Composition selon l'une quelconque des revendication 1 à 19, **caractérisée en ce qu'**elle est conditionnée sous forme de deux galets, un premier galet comprenant le système catalytique et un excipient et un second galet comprenant le précurseur de colorant, l'acide aminé à groupe thiol éventuel, et un excipient.

**33.** Composition selon l'une quelconque des revendications 30 à 32, **caractérisée en ce que** le ou les galets sont des galets effervescents.

**34.** Procédé pour révéler la coloration d'une composition de base peu ou pas colorée comprenant dans un milieu physiologiquement acceptable, au moins un précurseur de colorant choisi parmi les composés comportant au moins un cycle aromatique ayant au moins deux groupes hydroxyles portés par deux atomes de carbone consécutifs du cycle aromatique, éventuellement au moins un acide aminé comportant au moins un groupe thiol, **caractérisé en ce qu'**il consiste à ajouter à la composition de base, en présence d'oxygène, une quantité efficace d'un système catalytique comprenant un premier constituant choisi parmi les sels et oxydes de Mn(II) et/ou de Zn (II) et leurs mélanges et un second constituant choisi parmi les hydrogénocarbonates alcalins, les hydrogénocarbonates alcalino-terreux et leurs mélanges, les proportions du premier et du second constituant étant telles que :

$$\frac{[Mn(II)]}{[HCO_3]} \leq 1 \text{ avec } [Mn(II)] \neq 0$$

$$\frac{[Zn(II)]}{[HCO_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et $[HCO_3]$ représentent respectivement les concentrations molaires en Mn(II), Zn(II) et $HCO_3$ dans la composition et à mettre la composition de base additionnée du système catalytique en présence d'un milieu contenant de l'oxygène.

**35.** Procédé selon la revendication 34, **caractérisé en ce que** le rapport $\frac{[Mn(II)]}{[HCO_3]}$ varie de $10^{-5}$ à $10^{-1}$, de préférence de $10^{-3}$ à $10^{-2}$ et mieux est de l'ordre de $5.10^{-3}$.

**36.** Procédé selon la revendication 34, **caractérisé en ce que** le rapport $\frac{[Zn(II)]}{[HCO_3]}$ varie de $10^{-4}$ à < 1, de préférence de $10^{-3}$ à < 1, et mieux est de l'ordre de $5.10^{-1}$.

**37.** Procédé selon la revendication 34, **caractérisé en ce que** le rapport $\frac{[Mn(II) + Zn(II)]}{[HCO_3]}$ varie de $10^{-5}$ à $10^{-1}$, de préférence $10^{-3}$ à $10^{-2}$.

**38.** Procédé selon l'une quelconque des revendications 34 à 37, **caractérisé en ce que** le sel de Mn(II) et/ou le sel de Zn(II) sont choisis parmi les chlorure, fluorure, iodure, sulfate, acétate, phosphate, les sels d'acides carboxyliques et leurs mélanges.

**39.** Procédé selon l'une quelconque des revendications 34 à 38, **caractérisé en ce que** le sel de Mn(II) et/ou de Zn (II) est le chlorure.

**40.** Procédé selon la revendication 38, **caractérisé en ce que** les sels d'acides carboxyliques sont des sels d'acides carboxyliques hydroxylés.

**41.** Procédé selon la revendication 40, **caractérisé en ce que** le sel d'acide carboxylique hydroxylé est le gluconate.

**42.** Procédé selon l'une quelconque des revendications 34 à 41, **caractérisé en ce que** l'hydrogénocarbonate est choisi parmi l'hydrogénocarbonate de sodium, l'hydrogénocarbonate de potassium, l'hydrogénocarbonate de magnésium, l'hydrogénocarbonate de calcium et leurs mélanges.

**43.** Procédé selon l'une quelconque des revendications 34 à 42, **caractérisé en ce que** le cycle aromatique comportant

au moins deux groupes hydroxyle sur deux atomes de carbone consécutifs du précurseur de colorant est un cycle benzénique ou un cycle aromatique condensé.

**44.** Procédé selon l'une quelconque des revendications 34 à 43, **caractérisé en ce que** le précurseur de colorant est un composé de formule :

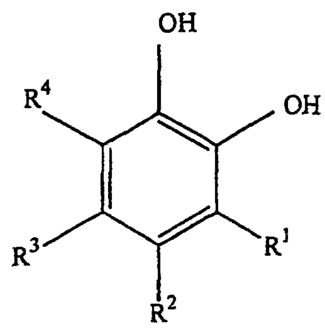

(I)

dans laquelle les substituants $R^1$ à $R^4$, identiques ou différents, représentent un atome d'hydrogène, un halogène, un radical hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical contenant éventuellement un ou plusieurs atomes de silicium, ou deux des substituants $R^1$ à $R^4$ peuvent former conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

**45.** Procédé selon l'une quelconque des revendications 34 à 43, **caractérisé en ce que** le précurseur de colorant est choisi parmi les flavanols, les flavonols, les anthocyanidines, les anthocyanines, les hydroxybenzoates, les flavones, les iridoïdes, ces composés pouvant être éventuellement osylés et/ou sous forme d'oligomères, les hydroxystilbènes éventuellement osylés, la 3,4-dihydroxyphénylalanine et ses dérivés, la 2,3-dihydroxyphénylalanine et ses dérivés, la 4,5-dihydroxyphénylalanine et ses dérivés, le 4,5-dihydroxyindole et ses dérivés, le 5,6-dihydroxyindole et ses dérivés, le 6,7-dihydroxyindole et ses dérivés, le 2,3-dihydroxyindole et ses dérivés, les dihydroxycinnamates, les hydroxycoumarines, les hydroxyisocoumarines, les hydroxycoumarones, les hydroxyisocoumarones, les hydroxychalcones, les hydroxychromones, les anthocyanes, les quinones, les hydroxyxanthones, et les mélanges de deux ou plus des composés précédents.

**46.** Procédé selon l'une quelconque des revendications 34 à 43, **caractérisé en ce que** le précurseur de colorant est choisi parmi les extraits de plantes, de fruits, d'agrumes, de légumes et leurs mélanges.

**47.** Procédé selon la revendication 46, **caractérisé en ce que** le précurseur de colorant est choisi parmi les extraits de thé, de raisin, de pomme, de banane, de pomme de terre et leurs mélanges.

**48.** Procédé selon l'une quelconque des revendications 34 à 47, **caractérisé en ce que** le précurseur de colorant est présent en une quantité d'au moins 10 micromoles par millilitre de composition.

**49.** Procédé selon l'une quelconque des revendications 34 à 48, **caractérisé en ce que** le ou les acides aminés comprenant au moins un groupe thiol sont choisis parmi les acides aminés ayant une fonction amine en position $\alpha$ par rapport à une fonction acide carboxylique.

**50.** Procédé selon la revendication 49, **caractérisé en ce que** le ou les acides aminés sont choisis parmi la cystéine et ses dérivés et le glutathion et ses dérivés.

**51.** Procédé selon l'une quelconque des revendications 34 à 50, **caractérisé en ce que** le rapport molaire du (ou des) acide(s) aminé(s) à groupe SH au(x) précurseurs) de colorant(s) varie de 0,01 à 5, de préférence de 0,05 à 2,5.

**52.** Procédé selon l'une quelconque des revendications 34 à 51, **caractérisé en ce que** le milieu physiologiquement

acceptable est un milieu solubilisant du précurseur de colorant et/ou de l'acide aminé à groupe thiol, de préférence à propriété bactériostatique.

**53.** Procédé selon l'une quelconque des revendications 34 à 52, **caractérisé en ce que** le milieu physiologiquement acceptable comprend un solvant ou un mélange de solvants du précurseur de colorant et/ou de l'acide aminé à groupe thiol.

**54.** Procédé selon la revendication 53, **caractérisé en ce que** le solvant est choisi parmi l'eau déminéralisée, les alcools, les éthers, le diméthylsulfoxyde, la N-méthylpyrrolidone, les cétones et leurs mélanges.

**55.** Procédé selon la revendication 54, **caractérisé en ce que** l'alcool est un alcanol ou un alcanediol.

**56.** Procédé selon la revendication 55, **caractérisé en ce que** le solvant est un mélange eau déminéralisée/alcool.

**57.** Procédé selon la revendication 56, **caractérisé en ce que** le mélange d'alcool représente jusqu'à 80% en poids du mélange, de préférence 1 à 50% en poids et mieux de 5 à 20% en poids.

**58.** Procédé selon l'une quelconque des revendications 34 à 57, **caractérisé en ce qu'**elle est exempte de tout agent de chélation du sel de Mn(II) et/ou Zn(II).

**59.** Procédé d'obtention d'une composition colorante, **caractérisé en ce qu'**il consiste à ajouter à de l'eau un galet comprenant dans un excipient physiologiquement acceptable au moins un précurseur de colorant, tel que défini à la revendication 1, éventuellement au moins un acide aminé comportant au moins un groupe thiol, de l'hydrogénocarbonate alcalin ou alcalino-terreux et éventuellement au moins un sel ou oxyde de Mn(II) et/ou de Zn(II) en quantités appropriées si l'eau ne contient pas de Mn(II) et / ou Zn(II) dans les proportions requises, de sorte que

$$\frac{[Mn(II)]\text{ et/ou }[\mathrm{Zn(II)}]}{[HCO_3]} \leq 1 \text{ avec } [\mathrm{Mn(II)}] \text{ et/ou } [\mathrm{Zn(II)}] \neq 0$$

**60.** Procédé d'obtention d'une composition colorante, **caractérisé en ce qu'**il consiste à ajouter à de l'eau un galet contenant dans un excipient physiologiquement acceptable au moins un précurseur de colorant, éventuellement au moins un acide aminé comportant au moins un groupe thiol, et un galet comprenant dans un excipient physiologiquement acceptable un système catalytique, le précurseur, l'acide aminé et le système catalytique étant tels que définis dans la revendication 1.

**61.** Procédé selon la revendication 59 ou 60, **caractérisé en ce que** le ou les galets sont des galets effervescents.

**62.** Procédé de coloration de la peau et/ou des matières kératiniques, **caractérisé en ce qu'**il consiste à appliquer sur la peau et/ou les matières kératiniques une couche d'une composition selon l'une quelconque des revendications 1 à 26.

**63.** Procédé selon la revendication 62, **caractérisé en ce que** la couche est obtenue en appliquant sur la peau et/ou les matières kératiniques un premier film d'une composition de base contenant le (ou les) précurseur(s) de colorant et éventuellement le ou les acides aminés comportant au moins un groupe thiol dans un milieu physiologiquement acceptable et en appliquant sur le premier film un second film d'une composition catalytique comprenant le système catalytique dans un milieu physiologiquement acceptable et vice-versa.

**64.** Procédé selon la revendication 62 ou 63, **caractérisé en ce que** les films sont appliqués par pulvérisation.

## Patentansprüche

**1.** Zusammensetzung zum Färben der Haut und/oder von Keratinfasern, **dadurch gekennzeichnet, dass** sie in einem physiologisch annehmbaren Medium folgendes umfasst: eine wirksame Menge wenigstens eines Farbstoffvorläufers, der aus den Verbindungen ausgewählt ist, die wenigstens einen aromatischen Ring umfassen, der wenigstens zwei Hydroxygruppen aufweist, die an zwei benachbarte Kohlenstoffatome des aromatischen Rings gebunden sind, gegebenenfalls eine wirksame Menge wenigstens einer Aminosäure, die wenigstens eine Thiolgruppe umfasst, und eine wirksame Menge eines Katalysatorsystems mit einem ersten Bestandteil, der aus den Salzen und Oxiden von Mn(II) und/oder Zn(II) und ihren Gemischen ausgewählt ist, und einem zweiten Bestandteil, der aus Alkalimetallhydrogencarbonaten, Erdalkalimetallhydrogencarbonaten und ihren Gemischen ausgewählt ist, wobei die Anteile des ersten und des zweiten Bestandteils so groß sind, dass:

$$\frac{[Mn(II)]}{[HCO_3]} \leq 1 \text{ mit } [Mn(II)] \neq 0$$

$$\frac{[Zn(II)]}{[HCO_3]} \leq 1 \text{ mit } [Zn(II)] \neq 0$$

$$\frac{[Mn(II) + Zn(II)]}{[HCO_3]} \leq 1 \text{ mit } [Mn(II)] \text{ und } [Zn(II)] \neq 0$$

wobei [Mn(II)], [Zn(II)] und [$HCO_3$] die molaren Konzentrationen von Mn(II), Zn(II) bzw. $HCO_3$ in der Zusammensetzung darstellen.

**2.** Zusammensetzung gemäß Anspruch 1, **dadurch gekennzeichnet, dass** das Verhältnis [Mn(II)]/[$HCO_3$] von $10^{-5}$ bis $10^{-1}$, vorzugsweise $10^{-3}$ bis $10^{-2}$, variiert und besonders bevorzugt in der Größenordnung von $5 \cdot 10^{-3}$ liegt.

**3.** Zusammensetzung gemäß Anspruch 1 oder 2, **dadurch gekennzeichnet, dass** das Verhältnis [Zn(II)]/[$HCO_3$] von $10^{-4}$ bis < 1, vorzugsweise $10^{-3}$ bis < 1, variiert und besonders bevorzugt in der Größenordnung von $5 \cdot 10^{-1}$ liegt.

**4.** Zusammensetzung gemäß einem der vorstehenden Ansprüche, **dadurch gekennzeichnet, dass** das Verhältnis [Mn(II) + Zn(II)]/[$HCO_3$] von $10^{-5}$ bis $10^{-1}$, vorzugsweise $10^{-3}$ bis $10^{-2}$, variiert.

**5.** Zusammensetzung gemäß einem der vorstehenden Ansprüche, **dadurch gekennzeichnet, dass** die Salze von Mn(II) und Zn(II) aus dem Chlorid, Fluorid, Iodid, Sulfat, Phosphat, Nitrat, Perchlorat, den Salzen von Carbonsäuren und ihren Gemischen ausgewählt sind.

**6.** Zusammensetzung gemäß Anspruch 5, **dadurch gekennzeichnet, dass** es sich bei dem Salz von Mn(II) und/oder Zn(II) um das Chlorid handelt.

**7.** Zusammensetzung gemäß Anspruch 5, **dadurch gekennzeichnet, dass** die Salze von Carbonsäuren Salze von Hydroxycarbonsäuren sind.

**8.** Zusammensetzung gemäß Anspruch 7, **dadurch gekennzeichnet, dass** es sich bei dem Salz der Hydroxycarbonsäure um Gluconat handelt.

**9.** Zusammensetzung gemäß einem der vorstehenden Ansprüche, **dadurch gekennzeichnet, dass** das Hydrogencarbonat aus Natriumhydrogencarbonat, Kaliumhydrogencarbonat, Magnesiumhydrogencarbonat, Calciumhydrogencarbonat und ihren Gemischen ausgewählt ist.

**10.** Zusammensetzung gemäß einem der vorstehenden Ansprüche, **dadurch gekennzeichnet, dass** der aromatische Ring, der wenigstens zwei Hydroxygruppen an zwei benachbarten Kohlenstoffatomen aufweist, des Farbstoffvorläufers ein benzolischer Ring oder ein kondensierter aromatischer Ring ist.

**11.** Zusammensetzung gemäß Anspruch 10, **dadurch gekennzeichnet, dass** der Farbstoffvorläufer eine Verbindung der folgenden Formel ist:

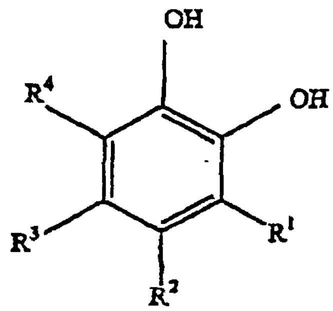

(I)

wobei die Substituenten $R^1$ bis $R^4$ gleich oder verschieden sind und folgendes darstellen: ein Wasserstoffatom, ein Halogen, eine Hydroxy-, Carboxy-, Alkylcarboxylatgruppe, gegebenenfalls substituiertes Amino, gegebenenfalls substituiertes lineares oder verzweigtes Alkyl, gegebenenfalls substituiertes lineares oder verzweigtes Alkenyl, gegebenenfalls substituiertes Cycloalkyl, Alkoxy, Alkoxyalkyl, Alkoxyaryl, wobei die Arylgruppe gegebenenfalls substituiert sein kann, Aryl, substituiertes Aryl, ein gegebenenfalls substituierter heterocyclischer Rest, ein Rest, der gegebenenfalls ein oder mehrere Siliciumatome enthält, oder zwei der Substituenten $R^1$ bis $R^4$ zusammen einen gesättigten oder ungesättigten Ring bilden können, der gegebenenfalls ein oder mehrere Heteroatome enthält und gegebenenfalls mit einem oder mehreren gesättigten oder ungesättigten Ringen, die gegebenenfalls ein oder mehrere Heteroatome enthalten, kondensiert ist.

**12.** Zusammensetzung gemäß einem der Ansprüche 1 bis 10, **dadurch gekennzeichnet, dass** der Farbstoffvorläufer ausgewählt ist aus Flavanolen, Flavonolen, Anthocyanidinen, Anthocyaninen, Hydroxybenzoaten, Flavonen, Iridoiden, wobei diese Verbindungen gegebenenfalls osyliert sein und/oder in Form von Oligomeren vorliegen können, gegebenenfalls osylierten Hydroxystilbenen, 3,4-Dihydroxyphenylalanin und seinen Derivaten, 2,3-Dihydroxyphenylalanin und seinen Derivaten, 4,5-Dihydroxyphenylalanin und seinen Derivaten, 4,5-Dihydroxyindol und seinen Derivaten, 5,6-Dihydroxyindol und seinen Derivaten, 6,7-Dihydroxyindol und seinen Derivaten, 2,3-Dihydroxyindol und seinen Derivaten, Dihydroxycinnamaten, Hydroxycumarinen, Hydroxyisocumarinen, Hydroxycumaronen, Hydroxyisocumaronen, Hydroxychalconen, Hydroxychromonen, Anthocyanen, Chinonen, Hydroxyxanthonen und den Gemischen von zwei oder mehreren der genannten Verbindungen.

**13.** Zusammensetzung gemäß einem der Ansprüche 1 bis 10, **dadurch gekennzeichnet, dass** der Farbstoffvorläufer aus Extrakten von Pflanzen, Früchten, Zitrusfrüchten, Gemüsen/Feldfrüchten und ihren Gemischen ausgewählt ist.

**14.** Zusammensetzung gemäß Anspruch 13, **dadurch gekennzeichnet, dass** der Farbstoffvorläufer aus Extrakten von Tee, Weintraube, Apfel, Banane, Kartoffel und ihren Gemischen ausgewählt ist.

**15.** Zusammensetzung gemäß einem der vorstehenden Ansprüche, **dadurch gekennzeichnet, dass** der Farbstoffvorläufer in einer Menge von wenigstens 10 $\mu$mol pro Milliliter der Zusammensetzung vorhanden ist.

**16.** Zusammensetzung gemäß einem der vorstehenden Ansprüche, **dadurch gekennzeichnet, dass** die Aminosäure oder -säuren, die wenigstens eine Thiolgruppe umfassen, aus Aminosäuren, die eine Aminofunktion in $\alpha$-Position in Bezug auf eine Carbonsäurefunktion aufweisen, ausgewählt sind.

**17.** Zusammensetzung gemäß Anspruch 16, **dadurch gekennzeichnet, dass** die Aminosäure oder -säuren aus Cystein und seinen Derivaten sowie Glutathion und seinen Derivaten ausgewählt sind.

**18.** Zusammensetzung gemäß einem der vorstehenden Ansprüche, **dadurch gekennzeichnet, dass** das Stoffmengenverhältnis der Aminosäure oder -säuren mit der SH-Gruppe zu dem bzw. den Farbstoffvorläufern von 0,001 bis 50, vorzugsweise 0,01 bis 5 und besonders bevorzugt 0,05 bis 2,5 variiert.

**19.** Zusammensetzung gemäß einem der vorstehenden Ansprüche, **dadurch gekennzeichnet, dass** es sich bei dem Farbstoffvorläufer um ein Dihydroxyphenylalanin oder ein Dihydroxyindol und bei der Aminosäure um Cystein oder Glutathion handelt.

**20.** Zusammensetzung gemäß einem der vorstehenden Ansprüche, **dadurch gekennzeichnet, dass** das physiologisch annehmbare Medium ein Medium ist, das den Farbstoffvorläufer und die Aminosäure auflöst, vorzugsweise mit bakteriostatischer Eigenschaft.

**21.** Zusammensetzung gemäß einem der vorstehenden Ansprüche, **dadurch gekennzeichnet, dass** das physiologisch annehmbare Medium ein Lösungsmittel oder ein Gemisch von Lösungsmitteln für den Farbstoffvorläufer und die Aminosäure umfasst.

**22.** Zusammensetzung gemäß Anspruch 21, **dadurch gekennzeichnet, dass** das Lösungsmittel aus Wasser, Alkoholen, Ethern, Dimethylsulfoxid, N-Methylpyrrolidon, Ketonen und ihren Gemischen ausgewählt ist.

**23.** Zusammensetzung gemäß Anspruch 22, **dadurch gekennzeichnet, dass** der Alkohol ein Alkanol oder ein Alkandiol ist.

**24.** Zusammensetzung gemäß Anspruch 22, **dadurch gekennzeichnet, dass** das Lösungsmittel ein Gemisch von Wasser und Alkohol ist.

**25.** Zusammensetzung gemäß Anspruch 24, **dadurch gekennzeichnet, dass** der Alkohol bis zu 80 Gew.-%, vorzugsweise 1 bis 50 Gew.-% und besonders bevorzugt 5 bis 20 Gew.-% des Gemischs ausmacht.

**26.** Zusammensetzung gemäß einem der vorstehenden Ansprüche, **dadurch gekennzeichnet, dass** sie keinerlei Chelatisierungsmittel des Salzes von Mn(II) und/oder Zn(II) enthält.

**27.** Zusammensetzung gemäß einem der vorstehenden Ansprüche, **dadurch gekennzeichnet, dass** sie in Form von zwei getrennten Komponenten vorliegt, wobei eine erste Komponente das in einem physiologisch annehmbaren Medium gelöste Katalysatorsystem umfasst und eine zweite Komponente den in einem physiologisch annehmbaren Medium gelösten Farbstoffvorläufer und gegebenenfalls die Aminosäure mit der Thiolgruppe umfasst.

**28.** Zusammensetzung gemäß einem der Ansprüche 1 bis 26, **dadurch gekennzeichnet, dass** sie in Form eines Aerosols oder eines Pumpsystems ohne Luftnachfüllung konditioniert ist.

**29.** Zusammensetzung gemäß Anspruch 27, **dadurch gekennzeichnet, dass** sie in einem Pumpsystem mit zwei getrennten Kammern konditioniert ist, wobei jede der Komponenten getrennt in einer der zwei Kammern enthalten ist.

**30.** Zusammensetzung gemäß einem der Ansprüche 1 bis 19, **dadurch gekennzeichnet, dass** sie in Form einer Tablette konditioniert ist.

**31.** Zusammensetzung gemäß Anspruch 30, **dadurch gekennzeichnet, dass** die Tablette eine Trägersubstanz umfasst, die Zitronensäure und/oder Weinsäure in einer substöchiometrischen Menge in Bezug auf das Alkalimetallund/oder Erdalkalimetallhydrogencarbonat enthält.

**32.** Zusammensetzung gemäß einem der Ansprüche 1 bis 19, **dadurch gekennzeichnet, dass** sie in Form von zwei Tabletten konditioniert ist, wobei eine erste Tablette das Katalysatorsystem und eine Trägersubstanz umfasst und eine zweite Tablette den Farbstoffvorläufer und gegebenenfalls die Aminosäure mit der Thiolgruppe sowie eine Trägersubstanz umfasst.

**33.** Zusammensetzung gemäß einem der Ansprüche 30 bis 32, **dadurch gekennzeichnet, dass** die Tablette oder Tabletten Brausetabletten sind.

**34.** Verfahren zum Sichtbarmachen der Färbung einer wenig oder nicht gefärbten Grundzusammensetzung, die in einem physiologisch annehmbaren Medium folgendes umfasst: wenigstens einen Farbstoffvorläufer, der aus den Verbindungen ausgewählt ist, die wenigstens einen aromatischen Ring umfassen, der wenigstens zwei Hydroxygruppen aufweist, die an zwei benachbarte Kohlenstoffatome des aromatischen Rings gebunden sind, gegebenenfalls wenigstens eine Aminosäure, die wenigstens eine Thiolgruppe umfasst, **dadurch gekennzeichnet, dass** es folgendes umfasst: die Zugabe einer wirksamen Menge eines Katalysatorsystems in Gegenwart von Sauerstoff zu der Grundzusammensetzung, wobei das Katalysatorsystem einen ersten Bestandteil, der aus den Salzen und Oxiden von Mn(II) und/oder Zn(II) und ihren Gemischen ausgewählt ist, und einen zweiten Bestandteil, der aus Alkalimetallhydrogencarbonaten, Erdalkalimetallhydrogencarbonaten und ihren Gemischen ausgewählt ist, umfasst, wobei die Anteile des ersten und des zweiten Bestandteils so groß sind, dass:

$$\frac{[Mn(II)]}{[HCO_3]} \leq 1 \text{ mit } [Mn(II)] \neq 0$$

$$\frac{[Zn(II)]}{[HCO_3]} \leq 1 \text{ mit } [Zn(II)] \neq 0$$

$$\frac{[Mn(II) + Zn(II)]}{[HCO_3]} \leq 1 \text{ mit } [Mn(II)] \text{ und } [Zn(II)] \neq 0$$

wobei [Mn(II)], [Zn(II)] und [$HCO_3$] die molaren Konzentrationen von Mn(II), Zn(II) bzw. $HCO_3$ in der Zusammensetzung darstellen; und
das In-Kontakt-Bringen der mit dem Katalysatorsystem versetzten Grundzusammensetzung mit einem Medium, das Sauerstoff enthält.

**35.** Verfahren gemäß Anspruch 34, **dadurch gekennzeichnet, dass** das Verhältnis [Mn(II)]/[$HCO_3$] von $10^{-5}$ bis $10^{-1}$, vorzugsweise $10^{-3}$ bis $10^{-2}$, variiert und besonders bevorzugt in der Größenordnung von $5 \cdot 10^{-3}$ liegt.

**36.** Verfahren gemäß Anspruch 34, **dadurch gekennzeichnet, dass** das Verhältnis [Zn(II)]/[$HCO_3$] von $10^{-4}$ bis < 1, vorzugsweise $10^{-3}$ bis < 1, variiert und besonders bevorzugt in der Größenordnung von $5 \cdot 10^{-1}$ liegt.

**37.** Verfahren gemäß Anspruch 34, **dadurch gekennzeichnet, dass** das Verhältnis [Mn(II) + Zn(II)]/[$HCO_3$] von $10^{-5}$ bis $10^{-1}$, vorzugsweise $10^{-3}$ bis $10^{-2}$, variiert.

**38.** Verfahren gemäß einem der Ansprüche 34 bis 37, **dadurch gekennzeichnet, dass** das Salz von Mn(II) und/oder das Salz von Zn(II) aus dem Chlorid, Fluorid, Iodid, Sulfat, Acetat, Phosphat, den Salzen von Carbonsäuren und ihren Gemischen ausgewählt ist.

**39.** Verfahren gemäß einem der Ansprüche 34 bis 38, **dadurch gekennzeichnet, dass** es sich bei dem Salz von Mn (II) und/oder Zn(II) um das Chlorid handelt.

**40.** Verfahren gemäß Anspruch 38, **dadurch gekennzeichnet, dass** die Salze von Carbonsäuren Salze von Hydroxycarbonsäuren sind.

**41.** Verfahren gemäß Anspruch 40, **dadurch gekennzeichnet, dass** es sich bei dem Salz der Hydroxycarbonsäure um Gluconat handelt.

**42.** Verfahren gemäß einem der Ansprüche 34 bis 41, **dadurch gekennzeichnet, dass** das Hydrogencarbonat aus Natriumhydrogencarbonat, Kaliumhydrogencarbonat, Magnesiumhydrogencarbonat, Calciumhydrogencarbonat und ihren Gemischen ausgewählt ist.

**43.** Verfahren gemäß einem der Ansprüche 34 bis 42, **dadurch gekennzeichnet, dass** der aromatische Ring, der wenigstens zwei Hydroxygruppen an zwei benachbarten Kohlenstoffatomen aufweist, des Farbstoffvorläufers ein benzolischer Ring oder ein kondensierter aromatischer Ring ist.

**44.** Verfahren gemäß einem der Ansprüche 34 bis 43, **dadurch gekennzeichnet, dass** der Farbstoffvorläufer eine Verbindung der folgenden Formel ist:

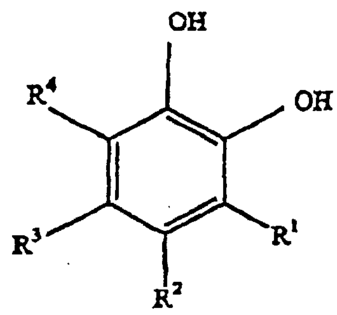

(I)

wobei die Substituenten $R^1$ bis $R^4$ gleich oder verschieden sind und folgendes darstellen: ein Wasserstoffatom, ein Halogen, eine Hydroxy-, Carboxy-, Alkylcarboxylatgruppe, gegebenenfalls substituiertes Amino, gegebenenfalls substituiertes lineares oder verzweigtes Alkyl, gegebenenfalls substituiertes lineares oder verzweigtes Alkenyl, gegebenenfalls substituiertes Cycloalkyl, Alkoxy, Alkoxyalkyl, Alkoxyaryl, wobei die Arylgruppe gegebenenfalls substituiert sein kann, Aryl, substituiertes Aryl, ein gegebenenfalls substituierter heterocyclischer Rest, ein Rest, der gegebenenfalls ein oder mehrere Siliciumatome enthält, oder zwei der Substituenten $R^1$ bis $R^4$ zusammen einen gesättigten oder ungesättigten Ring bilden können, der gegebenenfalls ein oder mehrere Heteroatome enthält und gegebenenfalls mit einem oder mehreren gesättigten oder ungesättigten Ringen, die gegebenenfalls ein oder mehrere Heteroatome enthalten, kondensiert ist.

**45.** Verfahren gemäß einem der Ansprüche 34 bis 43, **dadurch gekennzeichnet, dass** der Farbstoffvorläufer ausgewählt ist aus Flavanolen, Flavonolen, Anthocyanidinen, Anthocyaninen, Hydroxybenzoaten, Flavonen, Iridoiden, wobei diese Verbindungen gegebenenfalls osyliert sein und/oder in Form von Oligomeren vorliegen können, gegebenenfalls osylierten Hydroxystilbenen, 3,4-Dihydroxyphenylalanin und seinen Derivaten, 2,3-Dihydroxyphenylalanin und seinen Derivaten, 4,5-Dihydroxyphenylalanin und seinen Derivaten, 4,5-Dihydroxyindol und seinen Derivaten, 5,6-Dihydroxyindol und seinen Derivaten, 6,7-Dihydroxyindol und seinen Derivaten, 2,3-Dihydroxyindol und seinen Derivaten, Dihydroxycinnamaten, Hydroxycumarinen, Hydroxyisocumarinen, Hydroxycumaronen, Hydroxyisocumaronen, Hydroxychalconen, Hydroxychromonen, Anthocyanen, Chinonen, Hydroxyxanthonen und den Gemischen von zwei oder mehreren der genannten Verbindungen.

**46.** Verfahren gemäß einem der Ansprüche 34 bis 43, **dadurch gekennzeichnet, dass** der Farbstoffvorläufer aus Extrakten von Pflanzen, Früchten, Zitrusfrüchten, Gemüsen/Feldfrüchten und ihren Gemischen ausgewählt ist.

**47.** Verfahren gemäß Anspruch 46, **dadurch gekennzeichnet, dass** der Farbstoffvorläufer aus Extrakten von Tee, Weintraube, Apfel, Banane, Kartoffel und ihren Gemischen ausgewählt ist.

**48.** Verfahren gemäß einem der Ansprüche 34 bis 47, **dadurch gekennzeichnet, dass** der Farbstoffvorläufer in einer Menge von wenigstens 10 µmol pro Milliliter der Zusammensetzung vorhanden ist.

**49.** Verfahren gemäß einem der Ansprüche 34 bis 48, **dadurch gekennzeichnet, dass** die Aminosäure oder -säuren, die wenigstens eine Thiolgruppe umfassen, aus Aminosäuren, die eine Aminofunktion in α-Position in Bezug auf eine Carbonsäurefunktion aufweisen, ausgewählt sind.

**50.** Verfahren gemäß Anspruch 49, **dadurch gekennzeichnet, dass** die Aminosäure oder -säuren aus Cystein und seinen Derivaten sowie Glutathion und seinen Derivaten ausgewählt sind.

**51.** Verfahren gemäß einem der Ansprüche 34 bis 50, **dadurch gekennzeichnet, dass** das Stoffmengenverhältnis der Aminosäure oder -säuren mit der SH-Gruppe zu dem bzw. den Farbstoffvorläufern von 0,01 bis 50, vorzugsweise 0,05 bis 2,5, variiert.

**52.** Verfahren gemäß einem der Ansprüche 34 bis 51, **dadurch gekennzeichnet, dass** das physiologisch annehmbare Medium ein Medium ist, das den Farbstoffvorläufer und/oder die Aminosäure mit der Thiolgruppe auflöst, vorzugsweise mit bakteriostatischer Eigenschaft.

**53.** Verfahren gemäß einem der Ansprüche 34 bis 52, **dadurch gekennzeichnet, dass** das physiologisch annehmbare

Medium ein Lösungsmittel oder ein Gemisch von Lösungsmitteln für den Farbstoffvorläufer und/oder die Aminosäure mit der Thiolgruppe umfasst.

**54.** Verfahren gemäß Anspruch 53, **dadurch gekennzeichnet, dass** das Lösungsmittel aus entmineralisiertem Wasser, Alkoholen, Ethern, Dimethylsulfoxid, N-Methylpyrrolidon, Ketonen und ihren Gemischen ausgewählt ist.

**55.** Verfahren gemäß Anspruch 54, **dadurch gekennzeichnet, dass** der Alkohol ein Alkanol oder ein Alkandiol ist.

**56.** Verfahren gemäß Anspruch 55, **dadurch gekennzeichnet, dass** das Lösungsmittel ein Gemisch von entmineralisiertem Wasser und Alkohol ist.

**57.** Verfahren gemäß Anspruch 56, **dadurch gekennzeichnet, dass** das Alkoholgemisch bis zu 80 Gew.-%, vorzugsweise 1 bis 50 Gew.-% und besonders bevorzugt 5 bis 20 Gew.-% des Gemischs ausmacht.

**58.** Verfahren gemäß einem der Ansprüche 34 bis 57, **dadurch gekennzeichnet, dass** es keinerlei Chelatisierungsmittel des Salzes von Mn(II) und/oder Zn(II) beinhaltet.

**59.** Verfahren zur Gewinnung einer Färbezusammensetzung, **dadurch gekennzeichnet, dass** es die Zugabe einer Tablette zu Wasser umfasst, wobei die Tablette in einer physiologisch annehmbaren Trägersubstanz wenigstens einen Farbstoffvorläufer, wie er in Anspruch 1 definiert ist, gegebenenfalls wenigstens eine Aminosäure, die wenigstens eine Thiolgruppe umfasst, ein Alkali- oder Erdalkalimetallhydrogencarbonat und gegebenenfalls wenigstens ein Salz oder Oxid von Mn(II) und/oder Zn(II) in geeigneten Mengen, wenn das Wasser kein Mn(II) und/oder Zn (II) in den erforderlichen Anteilen enthält, umfasst, so dass:

$$\frac{[\text{Mn(II) und/oder Zn(II)}]}{[\text{HCO}_3]} \leq 1 \text{ mit } [\text{Mn(II)}] \text{ und/oder } [\text{Zn(II)}] \neq 0.$$

**60.** Verfahren zur Gewinnung einer Färbezusammensetzung, **dadurch gekennzeichnet, dass** es die Zugabe einer Tablette, die in einer physiologisch annehmbaren Trägersubstanz wenigstens einen Farbstoffvorläufer, gegebenenfalls wenigstens eine Aminosäure, die wenigstens eine Thiolgruppe umfasst, und einer Tablette, die in einer physiologisch annehmbaren Trägersubstanz ein Katalysatorsystem umfasst, zu Wasser umfasst, wobei der Vorläufer, die Aminosäure und das Katalysatorsystem wie in Anspruch 1 definiert sind.

**61.** Verfahren gemäß Anspruch 59 oder 60, **dadurch gekennzeichnet, dass** die Tablette oder Tabletten Brausetabletten sind.

**62.** Verfahren zum Färben der Haut und/oder von Keratinmaterial, **dadurch gekennzeichnet, dass** es das Auftragen einer Schicht einer Zusammensetzung gemäß einem der Ansprüche 1 bis 26 auf die Haut und/oder das Keratinmaterial umfasst.

**63.** Verfahren gemäß Anspruch 62, **dadurch gekennzeichnet, dass** die Schicht erhalten wird, indem man einen ersten Film aus einer Grundzusammensetzung, die den oder die Farbstoffvorläufer und gegebenenfalls die Aminosäure oder -säuren, die wenigstens eine Thiolgruppe umfassen, in einem physiologisch annehmbaren Medium enthält, auf die Haut und/oder die Keratinmaterialien aufträgt und auf den ersten Film einen zweiten Film aus einer Katalysatorzusammensetzung, die das Katalysatorsystem in einem physiologisch annehmbaren Medium enthält, aufträgt und umgekehrt.

**64.** Verfahren gemäß Anspruch 62 oder 63, **dadurch gekennzeichnet, dass** die Filme durch Besprühen aufgetragen werden.

## Claims

**1.** A composition for colouring skin and/or keratin fibres, **characterized in that** it comprises, in a physiologically acceptable medium, an efficient amount of at least one colouring agent precursor selected amongst the compounds comprising at least one aromatic cycle having at least two hydroxyl groups carried by two consecutive carbon atoms of the aromatic cycle, optionally an efficient amount of at least one amino acid comprising at least one thiol

group and an efficient amount of a catalytic system comprising a first component selected amongst salts and oxides of Mn(II) and/or Zn(II) and/or the mixtures thereof, and a second component selected amongst alkaline hydrogen carbonates, alkaline earth hydrogen carbonates and the mixtures thereof, the proportions of the first component and the second component being such that :

$$\frac{[Mn(II)]}{[HCO_3]} \leq 1 \text{ with } [Mn(II)] \neq 0$$

$$\frac{[Zn(II)]}{[HCO_3]} \leq 1 \text{ with } [Zn(II)] \neq 0$$

$$\frac{[Mn(II)+Zn(II)]}{[HCO_3]} \leq 1 \text{ with } [Mn(II)] \text{ and } [Zn(II)] \neq 0$$

where [Mn(II)], [Zn(II)] and $[HCO_3]$ represent respectively the Mn(II), Zn(II) and $HCO_3$ molar concentrations in the composition.

2. A composition according to claim 1, **characterized in that** the $\frac{[Mn(II)]}{[HCO_3]}$ ratio ranges from $10^{-5}$ to $10^{-1}$, preferably from $10^{-3}$ to $10^{-2}$ and more preferably is about $5.10^{-3}$.

3. A composition according to claim 1 or 2, **characterized in that** the $\frac{[Zn(II)]}{[HCO_3]}$ ranges from $10^{-4}$ to < 1, preferably from $10^{-3}$ to < 1 and more preferably is about $5.10^{-1}$.

4. A composition according to any one of the preceding claims, **characterized in that** the $\frac{[Mn(II)+Zn(II)]}{[HCO_3]}$ ratio ranges from $10^{-5}$ to $10^{-1}$, preferably from $10^{-3}$ to $10^{-2}$.

5. A composition according to any one of the preceding claims, **characterized in that** the Mn(II) and Zn(II) salts are selected amongst chloride, fluoride, iodide, sulfate, phosphate, nitrate, perchlorate, carboxylic acid salts and the mixtures thereof.

6. A composition according to claim 5, **characterized in that** the Mn(II) and/or Zn(II) salt is a chloride.

7. A composition according to claim 5, **characterized in that** the carboxylic acid salts are hydroxylated carboxylic acid salts.

8. A composition according to claim 7, **characterized in that** the hydroxylated carboxylic acid salt is a gluconate.

9. A composition according to any one of the preceding claims, **characterized in that** the hydrogen carbonate is selected amongst sodium hydrogen carbonate, potassium hydrogen carbonate, magnesium hydrogen carbonate, calcium hydrogen carbonate and the mixtures thereof.

10. A composition according to any one of the preceding claims, **characterized in that** the aromatic cycle comprising at least two hydroxyl groups on two consecutive carbon atoms of the colouring agent precursor is a benzene cycle or a condensed aromatic cycle.

11. A composition according to claim 10, **characterized in that** the colouring agent precursor is a compound of formula :

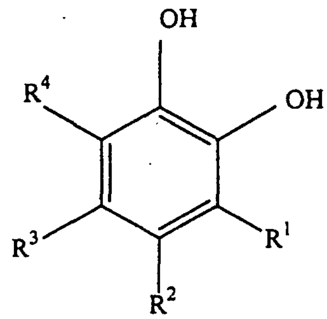

(I)

wherein the $R^1$ to $R^4$ sustituents, identical or different, represent a hydrogen atom, a halogen, hydroxyl, carboxyl, alkyl carboxylate radical, an optionally substituted amino radical, a linear or branched optionally substituted alkyl radical, a linear or branched optionally substituted alcenyl radical, a optionally substituted cycloalkyl radical, an alkoxy, alkoxyalkyl, alkoxyaryl radicals, the aryl group being able to be optionally substituted, aryl, substituted aryl, an optionally substituted heterocyclic radical, a radical containing optionally one or more silicon atoms, where two of the components $R^1$ to $R^4$ together form a saturated or unsaturated cycle optionally containing one or more heteroatoms and optionally condensed with one ore more saturated or unsaturated cycles optionally containing one or more heteroatoms.

**12.** A composition according to any one of claims 1 to 10, **characterized in that** the colouring agent precursor is selected amongst flavanols, flavonols, anthocyanidines, anthocyanines, hydroxybenzoates, flavones, iridoids, such compounds being optionally osylated and/or in the form of oligomers, hydroxystilbenes, optionally osylated, 3,4-dihydroxy-phenylalanine and the derivatives thereof, 2,3-dihydroxyphenylalanine and the derivatives thereof, 4,5-dihydroxyphenylalanine and the derivatives thereof, 4,5-dihydroxyindole and the derivatives thereof, 5,6-dihydroxyindole and the derivatives thereof, 6,7-dihydroxyindole and the derivatives thereof, 2,3-dihydroxyindole and the derivatives thereof, dihydroxycinnamates, hydroxycoumarins, hydroxyisocoumarins, hydroxycoumarones, hydroxy-isocoumarones, hydroxychalcones, hydroxychromones, anthocyans, quinones, hydroxyxanthones and the mixtures of two or more of the above-mentioned compounds.

**13.** A composition according to any one of claims 1 to 10, **characterized in that** the colouring agent precursor is selected amongst plant, fruit, citrus fruit, vegetable extracts, and the mixtures thereof.

**14.** A composition according to claim 13, **characterized in that** the colouring agent precursor is selected amongst tea, grape, apple, banana, potato extracts, and the mixtures thereof.

**15.** A composition according to any one of the preceding claims, **characterized in that** the colouring agent precursor is present in an amount of at least 10 micromoles per milliliter of the composition.

**16.** A composition according to any one of the preceding claims, **characterized in that** the amino acid(s) comprising at least one thiol group are selected amongst amino acids with an amine function in an $\alpha$ position relative to a carboxylic acid function.

**17.** A composition according to claim 16, **characterized in that** the amino acid(s) are selected amongst cysteine and the derivatives thereof and the glutathione and the derivatives thereof.

**18.** A composition according to any one of the preceding claims, **characterized in that** the amino acid(s) with a SH group/colouring agent precursor(s) molar ratio ranges from 0.001 to 50, preferably from 0.01 to 5, more preferably from from 0.05 to 2.5.

**19.** A composition according to any one of the preceding claims, **characterized in that** the colouring agent precursor is a dihydroxyphenylalanine or a dihydroxyindole and the amino acid, the cysteine or the glutatione.

**20.** A composition according to any one of the preceding claims, **characterized in that** the physiologically acceptable medium is a solubilizing medium of the colouring agent precursor and the amino acid, preferably having a bacteriostatic property.

**21.** A composition according to any one of the preceding claims, **characterized in that** the physiologically acceptable medium comprises a solvent or a mixture of solvents of the colouring agent precursor and the amino acid.

**22.** A composition according to claim 21, **characterized in that** the solvent is selected amongst water, alcohols, ethers, dimethylsulfoxide, N-methylpyrrolidone, ketones and the mixtures thereof.

**23.** A composition according to claim 22, **characterized in that** the alcohol is an alcanol or an alcanediol.

**24.** A composition according to claim 22, **characterized in that** the solvent is a water/alcohol mixture.

**25.** A composition according to claim 24, **characterized in that** the alcohol represents up to 80% wt of the mixture, preferably from 1 to 50% wt and more preferably from 5 to 20% wt.

**26.** A composition according to any one of the preceding claims, **characterized in that** it is free of any chelating agent of the Mn (II) and/or Zn (II) salt.

**27.** A composition according to any one of the preceding claims, **characterized in that** it has the form of two separate components, a first component comprising the catalytic system dissolved in a physiologically acceptable medium and a second component comprising the colouring agent precursor and optionally the amino acid having a thiol group dissolved in a physiologically acceptable medium.

**28.** A composition according to any one of claims 1 to 26, **characterized in that** it is packed in the form of an aerosol or a pump system with no air absorption.

**29.** A composition according to claim 27, **characterized in that** it is packed in a pump system with two discrete compartments, each of the components being contained separately in one of the two compartments.

**30.** A composition according to any one of claims 1 to 19, **characterized in that** it is packed in the form of a tablet.

**31.** A composition according to claim 30, **characterized in that** the tablet comprises an excipient containing citric and/or tartaric acid in a sub-stoichiometric amount relative to the alkaline and/or alkaline earth hydrogen carbonate.

**32.** A composition according to any one of claims 1 to 19, **characterized in that** it is packed in the form of two tablets, a first tablet comprising the catalytic system and an excipient and a second tablet comprising the colouring agent precursor, the amino acid with an optional thiol group, and an excipient.

**33.** A composition according to any one of claims 30 to 32, **characterized in that** the tablet(s) are effervescent tablets.

**34.** A method for revealing the colouring of a somewhat or a non coloured basic composition, comprising, in a physiologically acceptable medium, at least one colouring agent precursor selected amongst compounds having at least an aromatic cycle having at least two hydroxyl groups carried by two consecutive carbon atoms of the aromatic cycle, optionally at least one amino acid comprising at least one thiol group, **characterized in that** it involves adding to the basic composition, in the presence of oxygen, an efficient amount of a catalytic system comprising a first component selected amongst salts and oxides of Mn(II) and/or Zn(II) and the mixtures thereof and a second component selected amongst alkaline hydrogen carbonates, alkaline earth hydrogen carbonates and the mixtures thereof, the proportions of the first component and the second component being such that:

$$\frac{[Mn(II)]}{[HCO_3]} \leq 1 \text{ with } [Mn(II)] \neq 0$$

$$\frac{[Zn(II)]}{[HCO_3]} \leq 1 \text{ with } [Zn(II)] \neq 0$$

$$\frac{[Mn(II)+Zn(II)]}{[HCO_3]} \leq 1 \text{ with } [Mn(II)] \text{ and } [Zn(II)] \neq 0$$

where [Mn(II)], [Zn(II)] and [$HCO_3$] represent respectively the Mn(II), Zn(II) and $HCO_3$ molar concentrations in the composition, and involving bringing the basic composition with the catalytic system in the presence of an oxygen-containing medium.

**35.** A method according to claim 34, **characterized in that** the $\frac{[Mn(II)]}{[HCO_3]}$ ratio ranges from $10^{-5}$ to $10^{-1}$, preferably from $10^{-3}$ to $10^{-2}$ and more preferably is about $5.10^{-3}$.

**36.** A method according to claim 34, **characterized in that** the $\frac{[Zn(II)]}{[HCO_3]}$ ratio ranges from $10^{-4}$ to < 1, preferably from $10^{-3}$ to < 1 and more preferably is about $5.10^{-1}$.

**37.** A method according to claim 34, **characterized in that** the $\frac{[Mn(II)+Zn(II)]}{[HCO_3]}$ ratio ranges from $10^{-5}$ to $10^{-1}$, preferably from $10^{-3}$ to $10^{-2}$.

**38.** A method according to any one of claims 34 to 37, **characterized in that** the Mn(II) and Zn(II) salts are selected amongst chloride, fluoride, iodide, sulfate, acetate phosphate, carboxylic acid salts and the mixtures thereof.

**39.** A method according to any one of claims 34 to 38, **characterized in that** the Mn(II) and/or Zn(II) salt is a chloride.

**40.** A method according to claim 38, **characterized in that** the carboxylic acid salts are hydroxylated carboxylic acid salts.

**41.** A method according to claim 40, **characterized in that** the hydroxylated carboxylic acid salt is a gluconate.

**42.** A method according to any one of claims 34 to 41, **characterized in that** the hydrogen carbonate is selected amongst sodium hydrogen carbonate, potassium hydrogen carbonate, magnesium hydrogen carbonate, calcium hydrogen carbonate and the mixtures thereof.

**43.** A method according to any one of claims 34 to 42, **characterized in that** the aromatic cycle comprising at least two hydroxyl groups on two consecutive carbon atoms of the colouring agent precursor is a benzene cycle or a condensed aromatic cycle.

**44.** A method according to any one of claims 34 to 43, **characterized in that** the colouring agent precursor is a compound of formula :

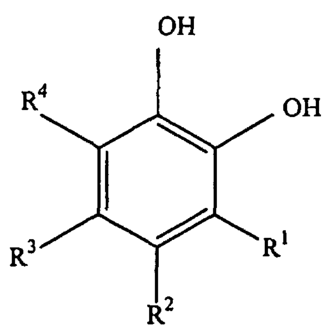

(I)

wherein the $R^1$ to $R^4$ substituents, identical or different, represent a hydrogen atom, a halogen, a hydroxyl, carboxyl, alkyl carboxylate radical, an optionally substituted amino radical, a linear or branched optionally substituted alkyl radical, a linear or branched optionally substituted alcenyl radical, a optionally substituted cycloalkyl radical, an alkoxy, alkoxyalkyl, alkoxyaryl, the aryl group being able to be optionally substituted, aryl, substituted aryl, an optionally substituted heterocyclic radical, a radical optionally containing one or more silicon atoms, or two of the components $R^1$ to $R^4$ are able to form together a saturated or unsaturated cycle optionally containing one or more heteroatoms and optionally condensed with one or more saturated or unsaturated cycles optionally containing one or more heteroatoms.

**45.** A method according to any one of claims 34 to 43, **characterized in that** the colouring agent precursor is selected amongst flavanols, flavonols, anthocyanidines, anthocyanines, hydroxybenzoates, flavones, iridoids, such compounds being optionally osylated and/or in the form of oligomers, hydroxystilbenes, optionally osylated, 3,4-dihydroxy-phenylalanine and the derivatives thereof, 2,3-dihydroxyphenylalanine and the derivatives thereof, 4,5-dihydroxyphenylalanine and the derivatives thereof, 4,5-dihydroxyindole and the derivatives thereof, 5,6-dihydroxyindole and the derivatives thereof, 6,7-dihydroxyindole and the derivatives thereof, 2,3-dihydroxyindole and the derivatives thereof, dihydroxycinnamates, hydroxycoumarins, hydroxyisocoumarins, hydroxycoumarones, hydroxyisocoumarones, hydroxychalcones, hydroxychromones, anthocyans, quinones, hydroxyxanthones and the mixtures of two or more of the above-mentioned compounds.

**46.** A method according to any one of claims 34 to 43, **characterized in that** the colouring agent precursor is selected amongst plant, fruit, citrus fruit, vegetable extracts, and the mixtures thereof.

**47.** A method according to claim 46, **characterized in that** the colouring agent precursor is selected amongst tea, grape, apple, banana, potato extracts, and the mixtures thereof.

**48.** A method according to any one of claims 34 to 47, **characterized in that** the the colouring agent precursor is present in an amount of at least 10 micromoles per milliliter of the composition.

**49.** A method according to any one of claims 34 to 48, **characterized in that** the amino acid(s) comprising at least one thiol group are selected amongst amino acids with an amine function in an $\alpha$ position relative to a carboxylic acid function.

**50.** A method according to claim 49, **characterized in that** the amino acid(s) are selected amongst cysteine and the derivatives thereof and the glutathione and the derivatives thereof.

**51.** A method according to any one of claims 34 to 50, **characterized in that** the amino acid(s) with a SH group/colouring agent precursor(s) molar ratio ranges from 0.01 to 5, preferably from 0.05 to 2.5.

**52.** A method according to any one of claims 34 to 51, **characterized in that** the physiologically acceptable medium is a solubilizing medium of the colouring agent precursor and/or the amino acid with a thiol group, preferably having a bacteriostatic property.

**53.** A method according to any one of claims 34 to 52, **characterized in that** the physiologically acceptable medium comprises a solvent or a mixture of solvents of the colouring agent precursor and/or the amino acid with a thiol group.

**54.** A method according to claim 53, **characterized in that** the solvent is selected amongst demineralized water, alcohols, ethers, dimethylsulfoxide, N-methylpyrrolidone, ketones and the mixtures thereof.

**55.** A method according to claim 54, **characterized in that** the alcohol is an alcanol or an alcanediol.

**56.** A method according to claim 55, **characterized in that** the solvent is a demineralized water/alcohol mixture.

**57.** A method according to claim 56, **characterized in that** the alcohol blend represents up to 80% wt of the mixture, preferably from 1 to 50% wt and more preferably from 5 to 20% wt.

**58.** A method according to any one of claims 34 to 57, **characterized in that** the composition is free of any chelating agent of the Mn (II) and/or Zn (II) salt.

**59.** A method for obtaining a colouring composition, **characterized in that** it involves adding to water a tablet comprising in a physiologically acceptable excipient at least one colouring agent precursor, such as defined in claim 1, optionally at least one amino acid comprising at least one thiol group, alkaline or alkaline earth hydrogen carbonate and optionally at least one salt or Mn(II) and/or Zn(II) oxide in suitable amounts if water does not contain any Mn(II) and/or Zn(II) in the required proportions, so that :

$$\frac{[Mn(II) and/or \mathrm{Zn}(II)]}{[HCO_3]} \leq 1 \text{ with [Mn(II)] and/or [Zn(II)]} \neq 0$$

**60.** A method for obtaining a colouring composition, **characterized in that** it involves adding to water a tablet comprising in a physiologically acceptable excipient at least one colouring agent precursor, optionally at least one amino acid comprising at least one thiol group, and a tablet comprising in a physiologically acceptable excipient a catalytic system, the precursor, the amino acid and the catalytic system being such as defined in claim 1.

**61.** A method according to claim 59 or 60, **characterized in that** the tablet(s) are effervescent tablets.

**62.** A method for colouring skin and/or keratin fibres, **characterized in that** it involves applying on skin and/or on keratin fibres a layer of a composition according to any one of claims 1 to 26.

**63.** A method according to claim 62, **characterized in that** the layer is obtained by applying on skin and/or on keratin fibres a first film of a basic composition containing the colouring agent precursor(s) and optionally the amino acid (s) comprising at least one thiol group in a physiologically acceptable medium, and applying on the first film a second film of a catalytic composition comprising the catalytic system in a physiologically acceptable medium or vice-versa.

**64.** A method according to claim 62 or 63, **characterized in that** the films are applied by spraying.